# EUROPEAN PATENT APPLICATION

(11) **EP 2 527 467 A2**
(43) Date of publication of application: **28.11.2012**
(21) Application number: 12154822.6
(22) Date of filing: 11.04.2008
(51) Int. Cl.: C12Q 1/68

(54) **Biomarkers and methods for determining sensitivity to vascular endothelial growth factor receptor-2 modulators**

(30) Priority: 13.04.2007 US 911547 P
(62) Divisional of application: 08745560.6
(71) Applicant: Bristol-Myers Squibb Company, Princeton, NJ 08543-4000 (US)
(72) Inventor: Parada, Jesus Platero, Washington Crossing, PA 18977 (US); Galbraith, Susan Mary, Newtown, PA 18940 (US)
(74) Representative: Reitstötter - Kinzebach

(57) **Abstract**

A method for predicting the likelihood a mammal will respond therapeutically to a method of treating cancer comprising administering a VEGFR-2 modulator or a dual VEGFR-2 / FGFR-1 modulator wherein the method comprises: (a) measuring in the mammal the level of a biomarker; (b) either comparing the level of the biomarker in the sample relative to a standard to permit assignment of the sample to either being a member of a biomarker positive class or a biomarker negative class, or comparing the level of the biomarker in the sample relative to a standard, wherein assignment of the mammal to the biomarker positive sample class or a determination that the mammal has an elevated level of the biomarker, indicates an increased or decreased likelihood the patient will respond therapeutically to the cancer treatment. Methods of predicting whether a mammal has received an efficacious dose of a VEGFR-2 modulator or a dual VEGFR-2 / FGFR-1 modulator is also disclosed, in addition to kits comprising these methods.

## Description

This application claims benefit to provisional application U.S. Serial No. 60/911,547, filed April 13, 2007, under 35 U.S.C. 119(e). The entire teachings of the referenced application are incorporated herein by reference.

### FIELD OF THE INVENTION

The present invention relates generally to the field of pharmacogenomics, and more specifically, to methods and procedures used to monitor response or determine sensitivity in patients to allow the identification of individualized genetic profiles which will aid in treating diseases and disorders.

### BACKGROUND OF THE INVENTION

Cancer is a disease with extensive histoclinical heterogeneity. Although conventional histological and clinical features have been correlated to prognosis, the same apparent prognostic type of tumors varies widely in its responsiveness to therapy and consequent survival of the patient.

New prognostic and predictive markers, which would facilitate an individualization of therapy for each patient, are needed to accurately predict patient response to treatments, such as small molecule or biological molecule drugs, in the clinic. The problem may be solved by the identification of new parameters that could better predict the patient's sensitivity to treatment. The classification of patient samples is a crucial aspect of cancer diagnosis and treatment. The association of a patient's response to a treatment with molecular and genetic markers can open up new opportunities for treatment development in non-responding patients, or distinguish a treatment's indication among other treatment choices because of higher confidence in the efficacy. Further, the pre-selection of patients who are likely to respond well to a medicine, drug, or combination therapy may reduce the number of patients needed in a clinical study or accelerate the time needed to complete a clinical development program (M. Cockett et al., Current Opinion in Biotechnology, 11:602-609 (2000)).

The ability to determine which patients are responding to anti-angiogenesis therapies (such as VEGFR-2 modulators) or predict drug sensitivity in patients is particularly challenging because drug responses reflect not only properties intrinsic to the target cells, but also a host's metabolic properties. Efforts to use genetic information to predict or monitor drug response have primarily focused on individual genes that have broad effects, such as the multidrug resistance genes mdr1 and mrp1 (P. Sonneveld, J. Intern. Med., 247:521-534 (2000)).

The development of microarray technologies for large scale characterization of gene mRNA expression pattern has made it possible to systematically search for molecular markers and to categorize cancers into distinct subgroups not evident by traditional histopathological methods (J. Khan et al., Cancer Res., 58:5009-5013 (1998); A.A. Alizadeh et al., Nature, 403:503-511 (2000); M. Bittner et al., Nature, 406:536-540 (2000); J. Khan et al., Nature Medicine, 7(6):673-679 (2001); and T.R. Golub et al., Science, 286:531-537 (1999); U. Alon et al., P.N.A.S. USA, 96:6745-6750 (1999)). Such technologies and molecular tools have made it possible to monitor the expression level of a large number of transcripts within a cell population at any given time (see, e.g., Schena et al., Science, 270:467-470 (1995); Lockhart et al., Nature Biotechnology, 14:1675-1680 (1996); Blanchard et al., Nature Biotechnology, 14:1649 (1996); U.S. Patent No. 5,569,588 to Ashby et al.).

Recent studies demonstrate that gene expression information generated by microarray analysis of human tumors can predict clinical outcome (L.J. van't Veer et al., Nature, 415:530-536 (2002); M. Shipp et al., Nature Medicine, 8(1):68-74 (2002); G. Glinsky et al., J. Clin. Invest., 113(6):913-923 (2004)). These findings bring hope that cancer treatment will be vastly improved by better predicting and monitoring the response of individual tumors to therapy.

PCT Application No. PCT/US2006/034201 provides biomarkers useful for identifying a mammal that will respond therapeutically to a method of treating cancer comprising administering a VEGFR-2 modulator.

Needed are new and alternative methods and procedures to determine drug sensitivity or monitor response in patients to allow the development of individualized diagnostics which are necessary to treat diseases and disorders based on patient response at a molecular level.

### SUMMARY OF THE INVENTION

The invention also provides methods and procedures for determining patient sensitivity or monitor response at the molecular level to one or more dual VEGFR-2 (vascular endothelial growth factor receptor 2) / FGFR1 (fibroblast growth factor receptor-1) modulators. The invention also provides methods of determining or predicting whether an individual requiring therapy for a disease state such as cancer will or will not respond to treatment, prior to administration of the treatment, wherein the treatment comprises administration of one or more dual VEGFR-2/FGFR1 modulators. The one or more dual VEGFR-2/FGFR1 modulators may be either small molecules, monoclonal antibodies, antisense molecules or their equivalents, RNAi molecules or their equivalents, etc.

The invention provides methods and procedures for determining patient sensitivity or monitor response at the molecular level to one or more vascular endothelial growth factor receptor 2 (VEGFR-2) modulators. The invention also provides methods of determining or predicting whether an individual requiring therapy for a disease state such as cancer will or will not respond to treatment, prior to administration of the treatment, wherein the treatment comprises administration of one or more VEGFR-2 modulators. The one or more VEGFR-2 modulators are compounds that can be selected from, for example, one or more VEGFR-2 specific ligands, one or more small molecule VEGFR-2 inhibitors, or one or more VEGFR-2 binding monoclonal antibodies.

The invention provides methods and procedures for determining patient sensitivity or monitor response at the molecular level to one or more FGFR-1 modulators. The invention also provides methods of determining or predicting whether an individual requiring therapy for a disease state such as cancer will or will not respond to treatment, prior to administration of the treatment, wherein the treatment comprises administration of one or more FGFR-1 modulators. The one or more FGFR-1 modulators are compounds that can be selected from, for example, one or more FGFR-1 specific ligands, one or more small molecule FGFR-1 inhibitors, or one or more FGFR-1 binding monoclonal antibodies.

In one aspect, the invention provides a method for predicting the likelihood a mammal will respond therapeutically to a method of treating cancer comprising administering a modulator, wherein the method comprises: (a) measuring in the mammal the level of FGF2; (b) exposing the mammal to said modulator selected from the group consisting of: a dual VEGFR-2/FGFR-1 modulator, VEGFR-2 modulator, and a FGFR-1 modulator; (c) following the exposing of step (b), measuring in the mammal the level of FGF2, wherein an increase in the level of FGF2 in step (c) compared to the level of the FGF2 measured in step (a) indicates an increased likelihood that the mammal will respond therapeutically to said method of treating cancer.

In another aspect, the biomarker can comprise FGF2 together with one or more additional biomarkers, such as, FGF 1, VEGFR-2, or collagen type IV.

A difference in the level of the biomarker that is sufficient to indicate whether the mammal will or will not respond therapeutically to the method of treating cancer can be readily determined by one of skill in the art using known techniques. The increase or decrease in the level of the biomarker can be correlated to determine whether the difference is sufficient to identify a mammal that will respond therapeutically. The difference in the level of the biomarker that is sufficient can, in one aspect, be predetermined prior to determining whether the mammal will respond therapeutically to the treatment. In one aspect, the difference in the level of the biomarker is a difference in the mRNA level (measured, for example, by RT-PCR or a microarray), such as at least about a two-fold difference, at least about a three-fold difference, or at least about a four-fold difference in the level of expression, or more. In another aspect, the difference in the level of the biomarker is determined at the protein level by mass spectral methods or by FISH or by IHC. In another aspect, the difference in the level of the biomarker refers to a p-value of <0.05 in Anova analysis. In yet another aspect, the difference is determined in an ELISA assay.

As used herein, respond therapeutically refers to the alleviation or abrogation of the cancer. This means that the life expectancy of an individual affected with the cancer will be increased or that one or more of the symptoms of the cancer will be reduced or ameliorated. The term encompasses a reduction in cancerous cell growth or tumor volume. Whether a mammal responds therapeutically can be measured by many methods well known in the art, such as PET imaging.

The mammal can be, for example, a human, rat, mouse, dog rabbit, pig sheep, cow, horse, cat, primate, or monkey.

The method of the invention can be, for example, an *in vitro* method wherein the step of measuring in the mammal the level of at least one biomarker comprises taking a biological sample from the mammal and then measuring the level of the biomarker(s) in the biological sample. The biological sample can comprise, for example, at least one of serum, whole fresh blood, peripheral blood mononuclear cells, frozen whole blood, fresh plasma, frozen plasma, urine, saliva, skin, hair follicle, bone marrow, or tumor tissue.

The level of the at least one biomarker can be, for example, the level of protein and/or mRNA transcript of the biomarker(s).

In another aspect, the invention provides a method for predicting the likelihood a mammal will respond therapeutically to a method of treating cancer comprising administering a modulator, wherein the method comprises: (a) exposing the mammal to the modulator; (b) following the exposing of step (a), measuring in the mammal the level of FGF2, wherein an increase in the level of FGF2 measured in step (b) compared to the level of the biomarker in a mammal that has not been exposed to said modulator indicates an increased likelihood that the mammal will respond therapeutically to said method of treating cancer, wherein said modulator is selected from the group consisting of: a dual VEGFR-2/FGFR-1 modulator, VEGFR-2 modulator, and a FGFR-1 modulator

In another aspect, the invention provides a method for determining whether a compound inhibits VEGFR-2 activity in a mammal, comprising: (a) exposing the mammal to the compound; and (b) following the exposing of step (a), measuring in the mammal the level of FGF2, wherein a difference in the level of FGF2 measured in step (b), compared to the level of FGF2 in a mammal that has not been exposed to said compound, indicates that the compound inhibits VEGFR-2 activity in the mammal.

In another aspect, the invention provides a method for determining whether a compound inhibits VEGFR-2 activity in a mammal, comprising: (a) exposing the mammal to the compound; and (b) following the exposing of step (a), measuring in the mammal the level of Collagen IV, wherein a difference in the level of Collagen IV measured in step (b), compared to the level of Collagen IV in a mammal that has not been exposed to said compound, indicates that the compound inhibits VEGFR-2 activity in the mammal.

In another aspect, the invention provides a method for predicting whether a patient suffering from cancer is receiving an efficacious dose of a cancer treatment comprising the administration of a VEGFR-2 modulator comprising the steps of: (a) measuring the level of Collagen IV in a sample from said patient; and (b) comparing the level of Collagen IV in said sample relative to a standard, wherein a decreased level of Collagen IV in said sample relative to said standard indicates an increased likelihood that the patient is receiving a therapeutically efficacious dose of said cancer treatment, whereas an increased or unchanged level of Collagen IV in said sample relative to said standard indicates a decreased likelihood that said patient has received a therapeutically efficacious dose of said cancer treatment. Said cancer treatment may be any of the treatments outlined herein which may include, for example, the administration of a VEGFR-2 modulator, or the administration of a dual VEGFR-2 / FGF modulator. In the instance where said patient is predicted to have not received an efficacious dose of said cancer treatment, a higher dose or dosing frequency, either alone or in combination with the administration of another treatment, of said treatment may be warranted.

In yet another aspect, the invention provides a method for determining whether a mammal has been exposed to a compound that inhibits VEGFR-2 activity, comprising: (a) exposing the mammal to the compound; and (b) following the exposing of step (a), measuring in the mammal the level of FGF2, wherein a difference in the level of FGF2 measured in step (b), compared to the level of FGF2 in a mammal that has not been exposed to said compound, indicates that the mammal has been exposed to a compound that inhibits VEGFR-2 activity.

In yet another aspect, the invention provides a method for determining whether a mammal has been exposed to a compound that inhibits VEGFR-2 activity, comprising: (a) exposing the mammal to the compound; and (b) following the exposing of step (a), measuring in the mammal the level of Collagen IV, wherein a difference in the level of Collagen IV measured in step (b), compared to the level of Collagen IV in a mammal that has not been exposed to said compound, indicates that the mammal has been exposed to a compound that inhibits VEGFR-2 activity.

In another aspect, the invention provides a method for determining whether a mammal is responding to a compound that inhibits VEGFR-2 activity, comprising: (a) exposing the mammal to the compound; and (b) following the exposing of step (a), measuring in the mammal the level of FGF2, wherein a difference in the level of FGF2 measured in step (b), compared to the level of FGF2 in a mammal that has not been exposed to said compound, indicates that the mammal is responding to the compound that inhibits VEGFR-2 activity.

In another aspect, the invention provides a method for determining whether a mammal is responding to a compound that inhibits VEGFR-2 activity, comprising: (a) exposing the mammal to the compound; and (b) following the exposing of step (a), measuring in the mammal the level of Collagen IV, wherein a difference in the level of Collagen IV measured in step (b), compared to the level of Collagen IV in a mammal that has not been exposed to said compound, indicates that the mammal is responding to the compound that inhibits VEGFR-2 activity.

In another aspect, the invention provides a method for determining whether a compound inhibits FGFR-1 activity in a mammal, comprising: (a) exposing the mammal to the compound; and (b) following the exposing of step (a), measuring in the mammal the level of FGF2, wherein a difference in the level of FGF2 measured in step (b), compared to the level of FGF2 in a mammal that has not been exposed to said compound, indicates that the compound inhibits FGFR-1 activity in the mammal.

In another aspect, the invention provides a method for determining whether a compound inhibits FGFR-1 activity in a mammal, comprising: (a) exposing the mammal to the compound; and (b) following the exposing of step (a), measuring in the mammal the level of Collagen IV, wherein a difference in the level of Collagen IV measured in step (b), compared to the level of Collagen IV in a mammal that has not been exposed to said compound, indicates that the compound inhibits FGFR-1 activity in the mammal.

In yet another aspect, the invention provides a method for determining whether a mammal has been exposed to a compound that inhibits FGFR-1 activity, comprising: (a) exposing the mammal to the compound; and (b) following the exposing of step (a), measuring in the mammal the level of FGF2, wherein a difference in the level of FGF2 measured in step (b), compared to the level of FGF2 in a mammal that has not been exposed to said compound, indicates that the mammal has been exposed to a compound that inhibits FGFR-1 activity.

In yet another aspect, the invention provides a method for determining whether a mammal has been exposed to a compound that inhibits FGFR-1 activity, comprising: (a) exposing the mammal to the compound; and (b) following the exposing of step (a), measuring in the mammal the level of Collagen IV, wherein a difference in the level of Collagen IV measured in step (b), compared to the level of Collagen IV in a mammal that has not been exposed to said compound, indicates that the mammal has been exposed to a compound that inhibits FGFR-1 activity.

In another aspect, the invention provides a method for determining whether a mammal is responding to a compound that inhibits FGFR-1 activity, comprising: (a) exposing the mammal to the compound; and (b) following the exposing of step (a), measuring in the mammal the level of FGF2, wherein a difference in the level of FGF2 measured in step (b), compared to the level of FGF2 in a mammal that has not been exposed to said compound, indicates that the mammal is responding to the compound that inhibits FGFR-1 activity.

In another aspect, the invention provides a method for determining whether a mammal is responding to a compound that inhibits FGFR-1 activity, comprising: (a) exposing the mammal to the compound; and (b) following the exposing of step (a), measuring in the mammal the level of Collagen IV, wherein a difference in the level of Collagen IV measured in step (b), compared to the level of Collagen IV in a mammal that has not been exposed to said compound, indicates that the mammal is responding to the compound that inhibits FGFR-1 activity.

As used herein, "responding" encompasses responding by way of a biological and cellular response, as well as a clinical response (such as improved symptoms, a therapeutic effect, or an adverse event), in a mammal

The invention also provides an isolated FGF2 biomarker, an isolated FGF1 biomarker, an isolated VEGFR-2 biomarker, and an isolated collagen type IV biomarker. The biomarkers of the invention include nucleotide and amino acid sequences of full-length FGF2, FGF1, VEGFR-2, and collagen type IV, as well as fragments and variants thereof.

The invention also provides a biomarker set comprising two or more biomarkers of the invention.

The invention also provides kits for determining or predicting whether a patient would be susceptible or resistant to a treatment that comprises one or more VEGFR-2 modulators, one or more FGFR-1 modulators, or one or more dual VEGFR-2/FGFR-1 modulators. The patient may have a cancer or tumor such as, for example, a colon cancer or tumor.

In one aspect, the kit comprises a suitable container that comprises one or more specialized microarrays, of the invention, one or more VEGFR-2 modulators, one or more FGFR-1 modulators, or one or more dual VEGFR-2 / FGFR-1 modulators for use in testing cells from patient tissue specimens or patient samples, and instructions for use. The kit may further comprise reagents or materials for monitoring the expression of a biomarker set at the level of mRNA or protein.

In another aspect, the invention provides a kit comprising two or more biomarkers.

In yet another aspect, the invention provides a kit comprising at least one of an antibody and a nucleic acid for detecting the presence of at least one of the biomarkers selected from FGF2, FGF1, VEGFR-2, and collagen type IV. In one aspect, the kit further comprises instructions for determining whether or not a mammal will respond therapeutically to a method of treating cancer comprising administering a compound that inhibits VEGFR-2 activity, FGFR-1 activity, or dual VEGFR-2/FGFR-1 activity.

The invention also provides screening assays for determining if a patient will be susceptible or resistant to treatment with one or more VEGFR-2 modulators, one or more FGFR-1 modulators, or one or more dual VEGFR-2 / FGFR-1 modulators.

The invention also provides a method of monitoring the treatment of a patient having a disease, wherein said disease is treated by a method comprising administering one or more VEGFR-2 modulators, one or more FGFR-1 modulators, or one or more dual VEGFR-2 / FGFR-1 modulators.

The invention also provides individualized genetic profiles which are necessary to treat diseases and disorders based on patient response at a molecular level.

The invention also provides specialized microarrays, e.g., oligonucleotide microarrays or cDNA microarrays, comprising one or more biomarkers having expression profiles that correlate with either sensitivity or resistance to one or more VEGFR-2 modulators, one or more FGFR-1 modulators, or one or more dual VEGFR-2 / FGFR-1 modulators.

The invention also provides antibodies, including polyclonal or monoclonal, directed against one or more biomarkers of the invention.

The invention will be better understood upon a reading of the detailed description of the invention when considered in connection with the accompanying figures.

### BRIEF DESCRIPTION OF THE FIGURES

The file of this patent contains at least one Figure executed in color. Copies of this patent with color Figure(s) will be provided by the Patent and Trademark Office upon request and payment of the necessary fee.

FIG. 1 (FIGS. 1A-1C) illustrates IHC results of collagen type IV.

FIG. 2 (FIGS. 2A-2B) illustrates IHC results of VEGFR-2.

FIG. 3 (FIGS. 3A-3C) illustrates IHC results of FGF1.

FIG. 4 (FIGS. 4A-4D) illustrates IHC results of FGF2.

FIG. 5 illustrates a boxplot for tumor percentage change from baseline versus FGF2 staining in responders and non-responders.

FIG. 6 illustrates Kaplan-Meier curves showing progression-free survival over time.

FIG. 7 illustrates results showing that brivanib modulated FGF2 growth factor gene expression levels whereas bevacizumab did not, while both brivanib and bevacizumab modulated VEGF gene expression levels. The results shown are from AFFYMETRIX® human arrays.

FIG. 8 illustrates results showing that brivanib inhibits FGF2-induced tumor cell line growth with much lower IC50 values than sunitinib *in vitro.*

FIG. 9 illustrates mRNA expression levels of FGFR1, FGF2, VEGFR2, and VEGF. in brivanib-sensitive and brivanib-resistant tumor cell lines.

FIG. 10 illustrates results showing that brivanib inhibits colon cancer cell growth directly when FGF2 is used to stimulate proliferation.

FIG. 11 illustrates results showing that brivanib inhibits ovarian cancer and pancreatic cancer cell line growth directly when FGF2 is used to stimulate proliferation.

FIG. 12 illustrates results showing that brivanib inhibited GEO tumor cell growth stimulated with FGF2 but not VEGF, compared to bevacizumab and sunitinib.

FIG 13 illustrates the effect of brivanib on phosphorylation of FGFR1, VEGFR2, ERK, and AKT using Western Blots.

FIG 14 illustrates expression analysis of 67 pathways in response to FGF2 stimulation. As shown, only the FGF pathway was significantly induced.

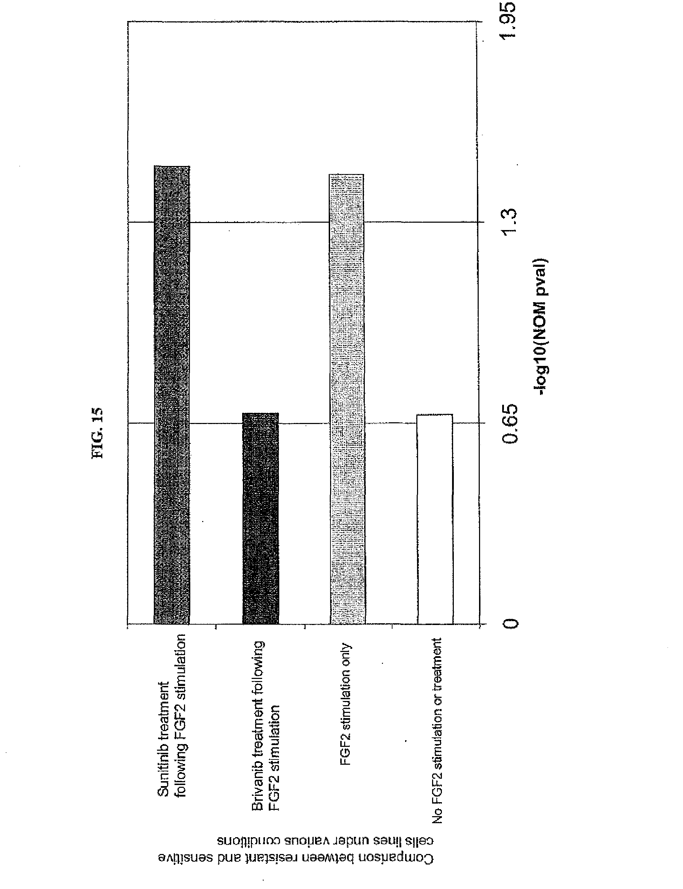

FIG 15 illustrates expression analysis of the FGF pathway in response to FGF2 stimulation after treatment with 1µm brivanib or 1µm sunitinib. As shown, FGF2-induced expression was abrograted only in the presence of only brivanib, but not sunitinib.

### DETAILED DESCRIPTION OF THE INVENTION

Multiple preclinical studies have demonstrated the important role VEGF plays in driving the angiogenic process through its cognate receptors, the VEGFR family of transmembrane protein tyrosine kinases. The VEGFR-2 signaling pathway, in particular, has been experimentally supported to be a major driver of tumor angiogenesis. The VEGFR-2 signaling pathway is a clinically validated pathway in cancer therapy based on the approval of AVASTIN® (bevacizumab), which indirectly inhibits this signaling pathway by preventing VEGF ligand binding. Brivanib has demonstrated potent inhibition of VEGFR-2 as well as inhibition of FGFR-1 and FGFR-2, another receptor family of protein tyrosine kinases underlying the angiogenic pathway and regulated by FGF growth factors. It is believed that by inhibiting multiple important angiogenesis pathways (VEGF and FGF), brivanib has shown to have a increased affect on the ability to inhibit tumor growth when compared to bevacizumab in a GEO colon cancer model.

This biomarker study has lead to the identification of a set of biomarkers that reflect anti-angiogenesis and anti-tumor activities at the molecular level for which brivanib has a greater affect relative to bevacizumab. The degree to which these biomarkers are affected is strongly associated with level of efficacy observed in this *in vivo* tumor model. These biomarkers could have important clinical implications in determining the optimal anti-angiogenesis therapy for cancer patients.

Identification of biomarkers that provide rapid and accessible readouts of efficacy, drug exposure, or clinical response is increasingly important in the clinical development of drug candidates. Embodiments of the invention include measuring changes in the levels of secreted proteins, or plasma biomarkers, which represent one category of biomarker. In one aspect, plasma samples, which represent a readily accessible source of material, serve as surrogate tissue for biomarker analysis.

The invention provides biomarkers that respond to the modulation of a specific signal transduction pathway and also correlate with VEGFR-2 modulator sensitivity or resistance. These biomarkers can be employed for predicting and monitoring response to one or more VEGFR-2 modulators. In one aspect, the biomarkers of the invention are selected from FGF2, FGF1, VEGFR-2, and collagen type IV, including both polynucleotide and polypeptide sequences. In another aspect, the biomarkers of the invention are nucleotide sequences that, due to the degeneracy of the genetic code, encodes for a polypeptide sequence provided in the sequence listing.

The biomarkers serve as useful molecular tools for predicting and monitoring response to VEGFR-2 modulators that affect VEGFR-2 activity or the VEGFR-2 signal transduction pathway.

@@

"About" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

Methods of measuring the level of any given marker described herein may be performed using methods well known in the art, which include, but are not limited to PCR; RT-PCR; FISH; IHC; immuno-detection methods; immunoprecipitation; Western Blots; ELISA; radioimmunoassays; PET imaging; HPLC; surface plasmon resonance, and optical spectroscopy; and mass spectrometry, among others.

The biomarkers of the invention may be quantified using any immunospecific binding method known in the art. The immunoassays which can be used include but are not limited to competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art (see, e.g., Ausubel et al., eds., Current Protocols in Molecular Biology, Vol. 1, John Wiley & Sons, Inc., New York (1994), which is incorporated by reference herein in its entirety). Exemplary immunoassays are described briefly below (but are not intended by way of limitation).

Immunoprecipitation protocols generally comprise lysing a population of cells in a lysis buffer such as RIPA buffer (1% NP-40 or Triton X- 100, 1% sodium deoxycholate, 0.1% SDS, 0.15 M NaCl, 0.01 M sodium phosphate at pH 7.2, 1% TRASYLOL®) supplemented with protein phosphatase and/or protease inhibitors (e.g., EDTA, PMSF, aprotinin, sodium vanadate), adding the antibody of interest (i.e., one directed to a biomarker of the present invention) to the cell lysate, incubating for a period of time (e.g., 1-4 hours) at 4° C, adding protein A and/or protein G SEPHAROSE® beads to the cell lysate, incubating for about an hour or more at 4° C, washing the beads in lysis buffer and resuspending the beads in SDS/sample buffer. The ability of the antibody of interest to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with SEPHAROSE® beads). For further discussion regarding immunoprecipitation protocols see, e.g., Ausubel et al., eds., Current Protocols in Molecular Biology, Vol. 1, John Wiley & Sons, Inc., New York at 10.16.1 (1994).

Western blot analysis generally comprises preparing protein samples, electrophoresis of the protein samples in a polyacrylamide gel (e.g., 8%- 20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 125I) diluted in blocking buffer, washing the membrane in wash buffer, and detecting the presence of the antigen. One of skill in the art would be knowledgeable as to the parameters that can be modified to increase the signal detected and to reduce the background noise. For further discussion regarding western blot protocols see, e.g., Ausubel et al., eds., Current Protocols in Molecular Biology, Vol. 1, John Wiley & Sons, Inc., New York at 10.8.1(1994).

ELISAs comprise preparing antigen, coating the well of a 96 well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art. For further discussion regarding ELISAs see, e.g., Ausubel et al., eds., Current Protocols in Molecular Biology, Vol. 1, John Wiley & Sons, Inc., New York at 11.2.1 (1994).

Alternatively, identifying the relative quantitation of the biomarker polypeptide(s) may be performed using tandem mass spectrometry; or single or multi dimensional high performance liquid chromatography coupled to tandem mass spectrometry. The method takes into account the fact that an increased number of fragments of an identified protein isolated using single or multi dimensional high performance liquid chromatography coupled to tandem mass spectrometry directly correlates with the level of the protein present in the sample. Such methods are well known to those skilled in the art and described in numerous publications, for example, 2-D Proteome Analysis Protocols, A.J. Link, ed., Humana Press (1999), ISBN: 0896035247; Mass Spectrometry of Proteins and Peptides, J.R. Chapman, ed., Humana Press (2000), ISBN: 089603609X.

As used herein the terms "modulate" or "modulates" or "modulators" refer to an increase or decrease in the amount, quality or effect of a particular activity, or the level of DNA, RNA, or protein detected in a sample.

### VEGFR-2 MODULATORS

As used herein, the term "VEGFR-2 modulator" is intended to mean a compound or drug that is a biological molecule or a small molecule that directly or indirectly modulates VEGFR-2 activity or the VEGFR-2 signal transduction pathway. Thus, compounds or drugs as used herein is intended to include both small molecules and biological molecules. Direct or indirect modulation includes activation or inhibition of VEGFR-2 activity or the VEGFR-2 signal transduction pathway. In one aspect, inhibition refers to inhibition of the binding of VEGFR-2 to a VEGFR-2 ligand such as, for example, VEGF. In another aspect, inhibition refers to inhibition of the kinase activity of VEGFR-2.

VEGFR-2 modulators include, for example, VEGFR-2 specific ligands, small molecule VEGFR-2 inhibitors, and VEGFR-2 monoclonal antibodies. In one aspect, the VEGFR-2 modulator inhibits VEGFR-2 activity and/or inhibits the VEGFR-2 signal transduction pathway. In another aspect, the VEGFR-2 modulator is a VEGFR-2 monoclonal antibody that inhibits VEGFR-2 activity and/or inhibits the VEGFR-2 signal transduction pathway.

VEGFR-2 modulators include biological molecules or small molecules.

VEGFR-2 modulators also include antisense and RNAi, molecules.

Biological molecules include all lipids and polymers of monosaccharides, amino acids, and nucleotides having a molecular weight greater than 450. Thus, biological molecules include, for example, oligosaccharides and polysaccharides; oligopeptides, polypeptides, peptides, and proteins; and oligonucleotides and polynucleotides. Oligonucleotides and polynucleotides include, for example, DNA and RNA.

Biological molecules further include derivatives of any of the molecules described above. For example, derivatives of biological molecules include lipid and glycosylation derivatives of oligopeptides, polypeptides, peptides, and proteins.

Derivatives of biological molecules further include lipid derivatives of oligosaccharides and polysaccharides, e.g., lipopolysaccharides. Most typically, biological molecules are antibodies, or functional equivalents of antibodies. Functional equivalents of antibodies have binding characteristics comparable to those of antibodies, and inhibit the growth of cells that express VEGFR-2. Such functional equivalents include, for example, chimerized, humanized, and single chain antibodies as well as fragments thereof.

Functional equivalents of antibodies also include polypeptides with amino acid sequences substantially the same as the amino acid sequence of the variable or hypervariable regions of the antibodies. An amino acid sequence that is substantially the same as another sequence, but that differs from the other sequence by means of one or more substitutions, additions, and/or deletions, is considered to be an equivalent sequence. Preferably, less than 50%, more preferably less than 25%, and still more preferably less than 10%, of the number of amino acid residues in a sequence are substituted for, added to, or deleted from the protein.

The functional equivalent of an antibody is preferably a chimerized or humanized antibody. A chimerized antibody comprises the variable region of a non-human antibody and the constant region of a human antibody. A humanized antibody comprises the hypervariable region (CDRs) of a non-human antibody. The variable region other than the hypervariable region, e.g., the framework variable region, and the constant region of a humanized antibody are those of a human antibody.

Suitable variable and hypervariable regions of non-human antibodies may be derived from antibodies produced by any non-human mammal in which monoclonal antibodies are made. Suitable examples of mammals other than humans include, for example, rabbits, rats, mice, horses, goats, or primates.

Functional equivalents further include fragments of antibodies that have binding characteristics that are the same as, or are comparable to, those of the whole antibody. Suitable fragments of the antibody include any fragment that comprises a sufficient portion of the hypervariable (i.e., complementarity determining) region to bind specifically, and with sufficient affinity, to VEGFR-2 tyrosine kinase to inhibit growth of cells that express such receptors.

Such fragments may, for example, contain one or both Fab fragments or the F(ab')2 fragment. Preferably, the antibody fragments contain all six complementarity determining regions of the whole antibody, although functional fragments containing fewer than all of such regions, such as three, four, or five CDRs, are also included.

In one aspect, the fragments are single chain antibodies, or Fv fragments. Single chain antibodies are polypeptides that comprise at least the variable region of the heavy chain of the antibody linked to the variable region of the light chain, with or without an interconnecting linker. Thus, Fv fragment comprises the entire antibody combining site. These chains may be produced in bacteria or in eukaryotic cells.

The antibodies and functional equivalents may be members of any class of immunoglobulins, such as IgG, IgM, IgA, IgD, or IgE, and the subclasses thereof.

In one aspect, the antibodies are members of the IgGl subclass. The functional equivalents may also be equivalents of combinations of any of the above classes and subclasses.

In one aspect, the VEGFR-2 antibody is CDP-791 (UCB). In another aspect, the VEGFR-2 antibody is IMC-1121b (ImClone Systems). In yet another aspect, the VEGFR-2 modulator is AVE-005 (VEGF trap, Regeneron Pharmaceuticals).

In addition to the biological molecules discussed above, the VEGFR-2 modulators useful in the invention may also be small molecules. Any molecule that is not a biological molecule is considered herein to be a small molecule. Some examples of small molecules include organic compounds, organometallic compounds, salts of organic and organometallic compounds, saccharides, amino acids, and nucleotides. Small molecules further include molecules that would otherwise be considered biological molecules, except their molecular weight is not greater than 450. Thus, small molecules may be lipids, oligosaccharides, oligopeptides, and oligonucleotides and their derivatives, having a molecular weight of 450 or less.

It is emphasized that small molecules can have any molecular weight. They are merely called small molecules because they typically have molecular weights less than 450. Small molecules include compounds that are found in nature as well as synthetic compounds. In one embodiment, the VEGFR-2 modulator is a small molecule that inhibits the growth of tumor cells that express VEGFR-2. In another embodiment, the VEGFR-2 modulator is a small molecule that inhibits the growth of refractory tumor cells that express VEGFR-2.

Numerous small molecules have been described as being useful to inhibit VEGFR-2.

In one aspect, the VEGFR-2 modulator is, brivanib, [(1*R*), 2*S*]-2-Aminopropionic acid 2-[4-(4-fluoro-2-methyl-1H-indol-5-yloxy)-5-methylpyrrolo [2,1-f][1,2,4]triazin-6-yloxy]-1-methylethyl ester having the structure (Compound I):

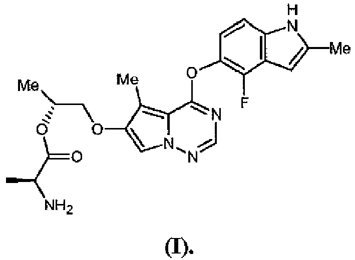

In another aspect, the VEGFR-2 modulator is selected from the compounds described in U.S. Patent No. 6,869,952, hereby incorporated by reference. In yet another aspect, the VEGFR-2 modulator is selected from the compounds described in PCT Publication No. WO00/71129 or WO2004/009601, hereby incorporated by reference.

In another aspect, the VEGFR-2 modulator is selected from Angiocept (BMS), CHIR-258 (Chiron), AZD-2171 (AstraZeneca, GW786034 (GlaxoSmithKline), AMG 706 (Amgen), BIBF 1120 (Boehringer Ingelheim), AE788 (Novartis), ZD6474 (AstraZeneca), BAY 43-9006 (Sorafenib, Bayer), SU11248 (Sutent, Pfizer), ranibizumab (Lucentis), sunitinib (Sutent), axitinib, temsirolimus, Vandetanib, SU5416, and Pazopanib (GW-786034).

### DUAL VEGFR-2 / FGFR-1 MODULATORS

As used herein, the term "dual VEGFR-2 / FGFR-1 modulator" is intended to mean a compound or drug that is a biological molecule or a small molecule that directly or indirectly modulates VEGFR-2 activity or the VEGFR signal transduction pathway, in addition to directly or indirectly modulating FGFR-1 activity or the FGF signal transduction pathway. Accordingly, a dual VEGFR-2 /FGFR-1 modulator may also encompass a biological molecule or a small molecule that inhibits VEGF, VEGFR-1, VEGFR-2, VEGFR-3, or any other VEGF or VEGF receptor within the VEGF pathway directly or indirectly, and also may inhibit FGF 1, FGF2, FGF3, FGF4, FGF5, FGF6, FGF7, FGF8, FGF9, FGF10, FGF11, FGF12, FGF13, FGF14, FGF15, FGF16, FGF17, FGF18, FGF19, FGF20, FGF21, FGF22, FGF23, FGFR-1, FGFR-2, FGFR-3, and/or FGFR-4 or any other FGF or FGF receptor within the FGF pathway directly or indirectly, known in the art. Thus, compounds or drugs as used herein is intended to include both small molecules and biological molecules. Direct or indirect modulation includes activation or inhibition of VEGFR-2 activity or the VEGFR-2 signal transduction pathway. Direct or indirect modulation includes activation or inhibition of FGFR-1 activity or the FGF signal transduction pathway. In one aspect, inhibition refers to inhibition of the binding of VEGFR-2 to a VEGFR-2 ligand such as, for example, VEGF. In another aspect, inhibition refers to inhibition of the kinase activity of VEGFR-2. Similarly, in one aspect, inhibition refers to inhibition of the binding of FGFR-1 to a FGFR-1 ligand such as, for example, FGF. In another aspect, inhibition refers to inhibition of the kinase activity of FGFR-1.

VEGFR-2 modulators are described elsewhere herein. FGFR-1 modulators include, for example, FGFR-1 specific ligands, small molecule FGFR-1 inhibitors, and FGFR-1 monoclonal antibodies. In one aspect, the FGFR-1 modulator inhibits FGFR-1 activity and/or inhibits the FGFR-1 signal transduction pathway. In another aspect, the FGFR-1 modulator is a FGFR-1 monoclonal antibody that inhibits FGFR-1 activity and/or inhibits the FGFR-1 signal transduction pathway.

FGFR-1 modulators include biological molecules or small molecules.

FGFR-1 modulators also include antisense and RNAi, molecules.

Biological molecules include all lipids and polymers of monosaccharides, amino acids, and nucleotides having a molecular weight greater than 450. Thus, biological molecules include, for example, oligosaccharides and polysaccharides; oligopeptides, polypeptides, peptides, and proteins; and oligonucleotides and polynucleotides. Oligonucleotides and polynucleotides include, for example, DNA and RNA.

Biological molecules further include derivatives of any of the molecules described above. For example, derivatives of biological molecules include lipid and glycosylation derivatives of oligopeptides, polypeptides, peptides, and proteins.

Derivatives of biological molecules further include lipid derivatives of oligosaccharides and polysaccharides, e.g., lipopolysaccharides. Most typically, biological molecules are antibodies, or functional equivalents of antibodies. Functional equivalents of antibodies have binding characteristics comparable to those of antibodies, and inhibit the growth of cells that express FGFR-1. Such functional equivalents include, for example, chimerized, humanized, and single chain antibodies as well as fragments thereof.

Functional equivalents of antibodies also include polypeptides with amino acid sequences substantially the same as the amino acid sequence of the variable or hypervariable regions of the antibodies. An amino acid sequence that is substantially the same as another sequence, but that differs from the other sequence by means of one or more substitutions, additions, and/or deletions, is considered to be an equivalent sequence. Preferably, less than 50%, more preferably less than 25%, and still more preferably less than 10%, of the number of amino acid residues in a sequence are substituted for, added to, or deleted from the protein.

The functional equivalent of an antibody is preferably a chimerized or humanized antibody. A chimerized antibody comprises the variable region of a non-human antibody and the constant region of a human antibody. A humanized antibody comprises the hypervariable region (CDRs) of a non-human antibody. The variable region other than the hypervariable region, e.g., the framework variable region, and the constant region of a humanized antibody are those of a human antibody.

Suitable variable and hypervariable regions of non-human antibodies may be derived from antibodies produced by any non-human mammal in which monoclonal antibodies are made. Suitable examples of mammals other than humans include, for example, rabbits, rats, mice, horses, goats, or primates.

Functional equivalents further include fragments of antibodies that have binding characteristics that are the same as, or are comparable to, those of the whole antibody. Suitable fragments of the antibody include any fragment that comprises a sufficient portion of the hypervariable (i.e., complementarity determining) region to bind specifically, and with sufficient affinity, to FGFR-1 tyrosine kinase to inhibit growth of cells that express such receptors.

Such fragments may, for example, contain one or both Fab fragments or the F(ab')2 fragment. Preferably, the antibody fragments contain all six complementarity determining regions of the whole antibody, although functional fragments containing fewer than all of such regions, such as three, four, or five CDRs, are also included.

In one aspect, the fragments are single chain antibodies, or Fv fragments. Single chain antibodies are polypeptides that comprise at least the variable region of the heavy chain of the antibody linked to the variable region of the light chain, with or without an interconnecting linker. Thus, Fv fragment comprises the entire antibody combining site. These chains may be produced in bacteria or in eukaryotic cells.

The antibodies and functional equivalents may be members of any class of immunoglobulins, such as IgG, IgM, IgA, IgD, or IgE, and the subclasses thereof.

In one aspect, the FGFR-1 antibody is the antibody disclosed by Beitz et al. (Cancer, 76(1):79-85 (1995)). In another aspect, the FGFR-1 antibody is 11A8 (Doukas et al., FASEB Journal, 13:1459-1466 (1999)). In yet another aspect, the FGFR-2 modulator is IMC-A1 (Imclone; Sun et al., Am. J. Physiol., Endocrinol Metab., November 28, 2006)).

In one aspect, the antibodies are members of the IgG1 subclass. The functional equivalents may also be equivalents of combinations of any of the above classes and subclasses.

In addition to the biological molecules discussed above, the FGFR-1 modulators useful in the invention may also be small molecules. Any molecule that is not a biological molecule is considered herein to be a small molecule. Some examples of small molecules include organic compounds, organometallic compounds, salts of organic and organometallic compounds, saccharides, amino acids, and nucleotides. Small molecules further include molecules that would otherwise be considered biological molecules, except their molecular weight is not greater than 450. Thus, small molecules may be lipids, oligosaccharides, oligopeptides, and oligonucleotides and their derivatives, having a molecular weight of 450 or less.

It is emphasized that small molecules can have any molecular weight. They are merely called small molecules because they typically have molecular weights less than 450. Small molecules include compounds that are found in nature as well as synthetic compounds. In one embodiment, the FGFR-1 modulator is a small molecule that inhibits the growth of tumor cells that express FGF. In another embodiment, the FGFR-1 modulator is a small molecule that inhibits the growth of refractory tumor cells that express FGFR-1.

Numerous small molecules have been described as being useful to inhibit both FGFR-1 and VEGFR-2. Non-limiting examples of such dual FGFR-1 and VEGFR-2 inhibitors include, for example, Compound I (Brivanib); soluble 7-substituted 3-(3,5-dimethoxyphenyl)-1,6-naphthyridin-2-amines and related ureas; 1-*t*-Butyl-3-(6-(3,5-dimethoxyphenyl)-2-(4-diethylaminobutylamino)-pyrido[2,3-d] pyrimidin-7-yl)urea (PD173074); substituted 3-[(4,5,6,7-tetrahydro-1H-indol-2-yl)methylene]-1,3-dihydroindol-2-ones (Sun et al., J. Med. Chem., 43:2655-2663 (2000)).

### BIOMARKERS AND BIOMARKER SETS

The invention includes individual biomarkers and biomarker sets having both diagnostic and prognostic value in disease areas in which signaling through VEGFR-2 or the VEGFR-2 pathway is of importance, e.g., in cancers or tumors, in immunological disorders, conditions or dysfunctions, or in disease states in which cell signaling and/or cellular proliferation controls are abnormal or aberrant. The biomarker sets comprise a plurality of biomarkers that highly correlate with resistance or sensitivity to one or more VEGFR-2 modulators.

The biomarkers and biomarker sets of the invention enable one to predict or reasonably foretell the likely effect of one or more VEGFR-2 modulators in different biological systems or for cellular responses. The biomarkers and biomarker sets can be used in *in vitro* assays of VEGFR-2 modulator response by test cells to predict *in vivo* outcome. In accordance with the invention, the various biomarkers and biomarker sets described herein, or the combination of these biomarker sets with other biomarkers or markers, can be used, for example, to predict and monitor how patients with cancer might respond to therapeutic intervention with one or more VEGFR-2 modulators.

A biomarker and biomarker set of cellular gene expression patterns correlating with sensitivity or resistance of cells following exposure of the cells to one or more VEGFR-2 modulators provides a useful tool for screening one or more tumor samples before treatment with the VEGFR-2 modulator. The screening allows a prediction of cells of a tumor sample exposed to one or more VEGFR-2 modulators, based on the expression results of the biomarker and biomarker set, as to whether or not the tumor, and hence a patient harboring the tumor, will or will not respond to treatment with the VEGFR-2 modulator.

The biomarker or biomarker set can also be used as described herein for monitoring the progress of disease treatment or therapy in those patients undergoing treatment for a disease involving a VEGFR-2 modulator.

The biomarkers also serve as targets for the development of therapies for disease treatment. Such targets may be particularly applicable to treatment of cancer, such as, for example, hepatocellular carcinoma, colorectal cancer (CRC), NSCLC, and metastatic breast cancer.

Indeed, because these biomarkers are differentially expressed in sensitive and resistant cells, their expression patterns are correlated with relative intrinsic sensitivity of cells to treatment with VEGFR-2 modulators. Accordingly, the biomarkers highly expressed in resistant cells may serve as targets for the development of new therapies for the tumors which are resistant to VEGFR-2 modulators, particularly VEGFR-2 inhibitors. The level of biomarker protein and/or mRNA can be determined using methods well known to those skilled in the art. For example, quantification of protein can be carried out using methods such as ELISA, 2-dimensional SDS PAGE, Western blot, immunoprecipitation, immunohistochemistry, fluorescence activated cell sorting (FACS), or flow cytometry. Quantification of mRNA can be carried out using methods such as PCR, array hybridization, Northern blot, in-situ hybridization, dot-blot, TAQMAN®, or RNAse protection assay.

### MICROARRAYS

The invention also includes specialized microarrays, e.g., oligonucleotide microarrays or cDNA microarrays, comprising one or more biomarkers, showing expression profiles that correlate with either sensitivity or resistance to one or more VEGFR-2 modulators. Such microarrays can be employed in *in vitro* assays for assessing the expression level of the biomarkers in the test cells from tumor biopsies, and determining whether these test cells are likely to be resistant or sensitive to VEGFR-2 modulators. For example, a specialized microarray can be prepared using all the biomarkers, or subsets thereof, as described herein. Cells from a tissue or organ biopsy can be isolated and exposed to one or more of the VEGFR-2 modulators. In one aspect, following application of nucleic acids isolated from both untreated and treated cells to one or more of the specialized microarrays, the pattern of gene expression of the tested cells can be determined and compared with that of the biomarker pattern from the control panel of cells used to create the biomarker set on the microarray. Based upon the gene expression pattern results from the cells that underwent testing, it can be determined if the cells show a resistant or a sensitive profile of gene expression. Whether or not the tested cells from a tissue or organ biopsy will respond to one or more of the VEGFR-2 modulators and the course of treatment or therapy can then be determined or evaluated based on the information gleaned from the results of the specialized microarray analysis.

### ANTIBODIES

The invention also includes antibodies, including polyclonal or monoclonal, directed against one or more of the polypeptide biomarkers. Such antibodies can be used in a variety of ways, for example, to purify, detect, and target the biomarkers of the invention, including both *in vitro* and *in vivo* diagnostic, detection, screening, and/or therapeutic methods.

### KITS

The invention also includes kits for determining or predicting whether a patient would be susceptible or resistant to a treatment that comprises one or more VEGFR-2 modulators. The patient may have a cancer or tumor such as, for example, a breast cancer or tumor. Such kits would be useful in a clinical setting for use in testing a patient's biopsied tumor or cancer samples, for example, to determine or predict if the patient's tumor or cancer will be resistant or sensitive to a given treatment or therapy with a VEGFR-2 modulator. The kit comprises a suitable container that comprises: one or more microarrays, e.g., oligonucleotide microarrays or cDNA microarrays, that comprise those biomarkers that correlate with resistance and sensitivity to VEGFR-2 modulators, particularly VEGFR-2 inhibitors; one or more VEGFR-2 modulators for use in testing cells from patient tissue specimens or patient samples; and instructions for use. In addition, kits contemplated by the invention can further include, for example, reagents or materials for monitoring the expression of biomarkers of the invention at the level of mRNA or protein, using other techniques and systems practiced in the art such as, for example, RT-PCR assays, which employ primers designed on the basis of one or more of the biomarkers described herein, immunoassays, such as enzyme linked immunosorbent assays (ELISAs), immunoblotting, e.g., Western blots, or in situ hybridization, and the like, as further described herein.

### APPLICATION OF BIOMARKERS AND BIOMARKER SETS

The biomarkers and biomarker sets may be used in different applications. Biomarker sets can be built to make predictions about the likely effect of any VEGFR-2 modulator in different biological systems. The various biomarkers and biomarkers sets described herein can be used, for example, as diagnostic or prognostic indicators in disease management, to predict how patients with cancer might respond to therapeutic intervention with compounds that modulate the VEGFR-2, and to predict how patients might respond to therapeutic intervention that modulates signaling through the entire VEGFR-2 regulatory pathway.

While the data described herein were generated in cell lines that are routinely used to screen and identify compounds that have potential utility for cancer therapy, the biomarkers have both diagnostic and prognostic value in other diseases areas in which signaling through VEGFR-2 or the VEGFR-2 pathway is of importance, e.g., in immunology, or in cancers or tumors in which cell signaling and/or proliferation controls have gone awry.

In accordance with the invention, cells from a patient tissue sample, e.g., a tumor or cancer biopsy, can be assayed to determine the expression pattern of one or more biomarkers prior to treatment with one or more VEGFR-2 modulators. Success or failure of a treatment can be determined based on the biomarker expression pattern of the cells from the test tissue (test cells), e.g., tumor or cancer biopsy, as being relatively similar or different from the expression pattern of a control set of the one or more biomarkers. Thus, if the test cells show a biomarker expression profile which corresponds to that of the biomarkers in the control panel of cells which are sensitive to the VEGFR-2 modulator, it is highly likely or predicted that the individual's cancer or tumor will respond favorably to treatment with the VEGFR-2 modulator. By contrast, if the test cells show a biomarker expression pattern corresponding to that of the biomarkers of the control panel of cells which are resistant to the VEGFR-2 modulator, it is highly likely or predicted that the individual's cancer or tumor will not respond to treatment with the VEGFR-2 modulator.

The invention also provides a method of monitoring the treatment of a patient having a disease treatable by one or more VEGFR-2 modulators. The isolated test cells from the patient's tissue sample, e.g., a tumor biopsy or blood sample, can be assayed to determine the expression pattern of one or more biomarkers before and after exposure to a VEGFR-2 modulator wherein, preferably, the VEGFR-2 modulator is a VEGFR-2 inhibitor. The resulting biomarker expression profile of the test cells before and after treatment is compared with that of one or more biomarkers as described and shown herein to be highly expressed in the control panel of cells that are either resistant or sensitive to a VEGFR-2 modulator. Thus, if a patient's response is sensitive to treatment by a VEGFR-2 modulator, based on correlation of the expression profile of the one or biomarkers, the patient's treatment prognosis can be qualified as favorable and treatment can continue. Also, if, after treatment with a VEGFR-2 modulator, the test cells don't show a change in the biomarker expression profile corresponding to the control panel of cells that are sensitive to the VEGFR-2 modulator, it can serve as an indicator that the current treatment should be modified, changed, or even discontinued. This monitoring process can indicate success or failure of a patient's treatment with a VEGFR-2 modulator and such monitoring processes can be repeated as necessary or desired.

The biomarkers of the invention can be used to predict an outcome prior to having any knowledge about a biological system. Essentially, a biomarker can be considered to be a statistical tool. Biomarkers are useful primarily in predicting the phenotype that is used to classify the biological system. In an embodiment of the invention, the goal of the prediction is to classify cancer cells as having an active or inactive VEGFR-2 pathway. Cancer cells with an inactive VEGFR-2 pathway can be considered resistant to treatment with a VEGFR-2 modulator.

### DOSING REGIMENS

For described herein, combination therapy refers to the administration of a VEGFR-2 modulator either alone or in combination with another VEGFR-2 modulator, a dual VEGFR-2 / FGFR-1 modulator either alone or in combination with a VEGFR-2 modulator or another dual VEGFR-2 / FGFR-1 modulator, the administration of [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester or a pharmaceutically acceptable salt, hydrate, or solvate thereof with a second therapy disclosed herein, including but not limited to a VEGFR-2 modulator or a dual VEGFR-2 / FGFR-1 modulator, or a pharmaceutically acceptable salt, hydrate, or solvate thereof. Such administration can involve concurrent (i.e., at the same time), prior, or subsequent administration of the second therapy with respect to the administration of [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester or salt, hydrate, or solvate thereof.

Treatment regimens can be based upon, for example, the predicted patient response to a therapy using the biomarkers described herein. For example, the invention encompasses measuring the FGF2 and/or Collagen IV levels of cells from an individual using, methods known in the art, who may suffer from, or is suffering from, a cancer or related disorder. For example, if a sample from a patient is determined to be FGF2(+), or if the patient sample shows elevated levels of FGF2 expression relative to a standard, the administration of a VEGFR-2 modulator or a dual VEGFR-2 / FGFR-1 modulator, or a pharmaceutically acceptable salt, hydrate, or solvate thereof, or the administration of [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester or salt, hydrate, or solvate thereof, may be warranted.

For those patients in which a sample is determined to be FGF2(-), or to have low or lower levels of FGF2 relative to a positive responding FGF2 reference sample, the administration of a combination therapy may be warranted, or an increased dose, or increased dosing frequency of any one of the following may be warranted: a VEGFR-2 modulator or a dual VEGFR-2 / FGFR-1 modulator, or a pharmaceutically acceptable salt, hydrate, or solvate thereof, or the administration of [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester or salt, hydrate, or solvate thereof, either alone or combination with another compound outlined herein. Additional treatment regimens are also contemplated by the present invention and are either disclosed herein or otherwise known in the art.

For those patients in which a sample is determined to not be responsive to the administration of a VEGFR-2 modulator or a dual VEGFR-2 / FGFR-1 modulator, or a pharmaceutically acceptable salt, hydrate, or solvate thereof, or the administration of [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-inethylethyl ester or salt, hydrate, or solvate thereof, on account of the level of Collagen IV remaining constant or at least not decreasing to a level deemed to represent a non-responding Collagen IV level, then the administration of a combination therapy may be warranted, or an increased dose, or increased dosing frequency of any one of the following may be warranted: a VEGFR-2 modulator or a dual VEGFR-2 / FGFR-1 modulator, or a pharmaceutically acceptable salt, hydrate, or solvate thereof, or the administration of [(1*R*), 2*S*]-2-Aminopropionic acid 2-[4-(4-fluoro-2-methyl-1H-indol-5-yloxy)-5-methylpyrrolo [2,1-f][1,2,4]triazin-6-yloxy]-1-methylethyl ester or salt, hydrate, or solvate thereof, either alone or combination with another compound outlined herein.

Additional treatment regimens are also contemplated by the present invention and are either disclosed herein or otherwise known in the art.

In one aspect, an increased level of [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester can be about 10, 20, 30, 40, 50, 60, 70, 80, 90, or 95% more than the typical [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester dose for a particular indication or for individual, or about 1.5x, 2x, 2.5x, 3x, 3.5x, 4x, 4.5x, 5x, 6x, 7x, 8x, 9x, or 10x more [(1*R*), 2*S*]-2-Aminopropionic acid 2-[4-(4-fluoro-2-methyl-1H-indol-5-yloxy)-5-methylpyirrolo[2,1-f][1,2,4]triazin-6-yloxy]-1-methylethyl ester than the typical [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester dose for a particular indication or for individual, or other therapy outlined herein. The same increased level also applies to any other VEGFR-2 modulator or dual VEGFR-2 / FGFR-1 modulator therapy.

A therapeutically effective amount of [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester or a pharmaceutically acceptable salt, hydrate, or solvate thereof can be orally administered. The actual dosage employed can be varied depending upon the requirements of the patient and the severity of the condition being treated. Determination of the proper dosage for a particular situation is within the skill of the art. The effective amount of [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester or a pharmaceutically acceptable salt, hydrate, or solvate thereof (and Compound I salt) can be determined by one of ordinary skill in the art, and includes exemplary dosage amounts for an adult human of from about 0.05 to about 100 mg/kg of body weight of [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester or a pharmaceutically acceptable salt, hydrate, or solvate thereof, per day, which can be administered in a single dose or in the form of individual divided doses, such as from about 1, 2, 3, 4, 5, or more times per day. In certain embodiments, [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester or a pharmaceutically acceptable salt, hydrate, or solvate thereof is administered 1, 2, 3, 4, 5, 6, 7, or 8 times per day up to a total of about 800 mg per day. Alternatively, it can be dosed at, for example, anywhere between about 15mg to 1000mg once daily, or about 15mg, about 60mg, about 180mg, about 320mg, about 800mg, or about 1000mg once daily, or anywhere between about 180mg to about 1000mg twice daily, or about 400mg twice daily. In addition, it can also be dosed at, for example about 200mg 1, 2, 3, 4, 5, 6, 7, or 8 times per day. It will be understood that the specific dose level and frequency of dosing for any particular subject can be varied and will depend upon a variety of factors including the activity of the specific compound employed, the metabolic stability and length of action of that compound, the species, age, body weight, general health, sex and diet of the subject, the mode and time of administration, rate of excretion, drug combination, and severity of the particular condition. Preferred subjects for treatment include animals, most preferably mammalian species such as humans, and domestic animals such as dogs, cats, and the like, subject to protein tyrosine kinase-associated disorders. The same also applies to any other VEGFR-2 modulator or dual VEGFR-2 / FGFR-1 modulator therapy, or any combination disclosed herein.

A method of determining the responsiveness of an individual suffering from cancer to a therapy, such as a VEGFR-2 modulator, a dual VEGFR-2 FGFR-1 modulator, and/or [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester, is disclosed herein. For example, an individual can be determined to be a positive responder (or cells from said individual would be expected to have a degree of sensitivity) to a certain kinase inhibitor based upon whether the patient sample is FGF2(+), if the patient sample shows an increased level of FGF2 relative to a standard level, and/or if the patient sample shows a corresponding decrease in the level of Collagen IV subsequent to the administration of a VEGFR-2 modulator, a dual VEGFR-2 / FGFR-1 modulator, or [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester. Similarly, an individual can be determined to be a negative responder (or cells from said individual would be expected to have a degree of resistance) to a certain kinase inhibitor based upon whether the patient sample is FGF2(-), if the patient sample shows an decreased level of FGF2 relative to a standard level, and/or if the patient sample fails to show a corresponding decrease in the level of Collagen IV subsequent to the administration of a VEGFR-2 modulator, a dual VEGFR-2 / FGFR-1 modulator, or [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester. Therefore, individuals suffering from a cancer whose sample /cells exhibit such a non-responsive phenotype are or would be expected to be a negative responder or at least a partially negative responder to a particular treatment regimen with a VEGFR-2 modulator, a dual VEGFR-2 / FGFR-1 modulator, or [(1*R*), 2*S*]-2-Aminopropionic acid 2-[4-(4-fluoro-2-methyl-1H-indol-5-yloxy)-5-methylpyrrolo [2,1-f][1,2,4]triazin-6-yloxy]-1-methylethyl ester or a pharmaceutically acceptable salt, hydrate, or solvate thereof but a positive responder to a more aggressive treatment regimen of a VEGFR-2 modulator, a dual VEGFR-2 /FGFR-1 modulator, or [(1*R*), 2*S*]-2-Aminopropionic acid 2-[4-{4-fluoro-2-methyl-1H-indol-5-yloxy)-5-methylpyrrolo[2,1-f][1,2,4]triazin-6-yloxy]-1-methylethyl ester or a pharmaceutically acceptable salt, hydrate, or solvate thereof or to combination therapy with another VEGFR-2 modulator, a dual VEGFR-2 / FGFR-1 modulator, or [(1*R*), 2*S*]-2-Aminopropionic acid 2-[4-(4-fluoro-2-methyl-1H-indol-5-yloxy)-5-methylpyrrolo [2,1-f][1,2,4]triazin-6-yloxy]-1-methylethyl ester or a pharmaceutically acceptable salt, hydrate, or solvate thereof and imatinib or other therapy.

A treatment regimen is a course of therapy administered to an individual suffering from cancer that can include treatment with one or more VEGFR-2 modulators, dual VEGFR-2 / FGFR-1 modulators, or [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester inhibitors, as well as other therapies such as radiation and/or other agents *(i.e.,* combination therapy). When more than one therapy is administered, the therapies can be administered concurrently or consecutively (for example, more than one kinase inhibitor can be administered together or at different times, on a different schedule). Administration of more than one therapies can be at different times (i.e., consecutively) and still be part of the same treatment regimen. Where more aggressive therapy is warranted, combination therapy or a more aggressive dosage or dosing regimen of N-(2-chloro-6-methylphenyl)-2-[[6-[4-(2-hydroxyethyl)-1-piperazinyl]-2-methyl-4-pyrimidinyl]amino]-5-thiazolecarboxamide or a pharmaceutically acceptable salt, hydrate, or solvate thereof, a treatment regimen can be established that includes treatment with the combination either as a monotherapy, or in combination with another kinase inhibitor, or in combination with another agent as disclosed herein. Additionally, the combination can be administered with radiation or other known treatments. Such other agents may include ERBITUX® (cetuximab), an epothilone, TAXOL®, paclitaxol, ixabepilone, a tubulin stabilizing agent (e.g., pacitaxol, epothilone, taxane, etc.), a farnysyl transferase inhibitor, 5-fluorouracil, leucovorin, irinotecan, FOLFIRI, oxaliplatin, FOLFOX, an anti-angiogensis therapy, etc.

In practicing the many aspects of the invention herein, biological samples can be selected from many sources such as tissue biopsy (including cell sample or cells cultured therefrom; biopsy of bone marrow or solid tissue, for example cells from a solid tumor), blood, blood cells (red blood cells or white blood cells), serum, plasma, lymph, ascetic fluid, cystic fluid, urine, sputum, stool, saliva, bronchial aspirate, CSF or hair. Cells from a sample can be used, or a lysate of a cell sample can be used. In certain embodiments, the biological sample is a tissue biopsy cell sample or cells cultured therefrom, for example, cells removed from a solid tumor or a lysate of the cell sample. In certain embodiments, the biological sample comprises tumor samples and/or blood cells.

Dosage regimens involving [(1*R*), 2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester useful in practicing the present invention are described in Garrett et al., (Journal of Clinical Oncology, 2007 ASCO Annual Meeting Proceedings, Vol. 25, No 18S (June 20 Supplement) (2007): 14018); which are hereby incorporated herein by reference in their entirety and for all purposes.

Pharmaceutical compositions for use in the present invention can include compositions comprising one or a combination of inhibitors of VEGFR-2, dual inhibitors of VEGFR-2 / FGFR-1, or [(1*R*), 2*S*]-2-Aminopropionic acid 2-[4-(4-fluoro-2-methyl-1H-indol-5-yloxy)-5-methylpyrrolo[2, 1-f][1,2,4]triazin-6-yloxy]-1-methylethyl ester in an effective amount to achieve the intended purpose. The determination of an effective dose of a pharmaceutical composition of the invention is well within the capability of those skilled in the art. A therapeutically effective dose refers to that amount of active ingredient which ameliorates the symptoms or condition. Therapeutic efficacy and toxicity can be determined by standard pharmaceutical procedures in cell cultures or experimental animals, for example the ED50 (the dose therapeutically effective in 50% of the population) and LD50 (the dose lethal to 50% of the population).

A "therapeutically effective amount" of an inhibitor of VEGFR-2 or a dual inhibitor of VEGFR-2 / FGFR-1 can be a function of the FGF2 status and/or responsiveness of the sample based upon the observed change in Collagen IV levels. For example, if a samples is deemed to be FGF2(+) and responsive based upon observed changes in Collagen IV subsequent to the administration of a treatment, the normal or prescribed dose for a patient may be sufficient to treat the patient. One skilled in the art will appreciate the difference in sensitivity of between a responsive and non-responsive sample, and/or a sensitive or resistant sample, and how such observations may be used to design a treatment regimen and determine a therapeutically effective dose accordingly. For a responsive, sensitive patient samples, therapeutically relevant doses of [(1*R*), 2*S*]-2-Aminopropionic acid 2-[4-(4-fluoro-2-methy-1H-indol-5-yloxy)-5-methylpyrrolo[2,1-f][1,2,4]triazin-6-yloxy]-1-methylethyl ester can be, for example, 0.9x, 0.8x, 0.7x, 0.6x, 0.5x, 0.4x, 0.3x, 0.2x, 0.1x, 0.09x, 0.08x, 0.07x, 0.06x, 0.05x, 0.04x, 0.03x, 0.02x, or 0.01x of the prescribed dose.

The following Exemplary Embodiments of specific aspects for carrying out the present invention are offered for illustrative purposes only, and are not intended to limit the scope of the present invention in any way.

### REFERENCES

1. J. Fargnoli et al., Proc. Amer. Assoc. Cancer Res., 46:Abstract #3033 (2005).
2. M. Ayers et al., Proc. Amer Assoc. Cancer Res., 48:Abstract #1618 (2007).
3. D.J. Jonker et al., 2007 ASCO Annual Meeting Proceedings Part I, Vol, 25, No. 18S (June 20 Supplement): Abstract # 3559 (2007).
4. K. Suzuki et al., Int. J. Urol., 12:152 (2005).
5. S. Javerzat et al., Trends Mol. Med., 8,:483 (2002).
6. Y. Shing et al., Science, 223:1296 (1984).
7. N. Yoshimura et al., Clin. Immunol. Immunopathol., 89:28 (1998).
8. L.A. Chandler et al., Int. J. Cancer, 81:451 (1999).
9. J.A. Takahashi et al., J. Neurosurg., 76:792 (1992).
10. R.A. Lindner et al., J. Clin. Invest., 85:2004 (1990).
11. H. Miyake et al., Cancer Res., 56:2440 (1996).
12. I. el-Hariry et al., J. Pathol., 181:39 (1997),
13. T. Ohta et al., Br. J. Cancer, 72:824 (1995).
14. T. Ueba et al., Proc. Natl. Acad. Sci. U.S.A., 91:9009 (1994).
15. Y. Yamanaka et al., Cancer Res., 53:5289 (1993).
16. M. Ayers et al., Cancer Res., 67:6899 (2007).
17. N. Hiraoka et al., Cell, 95:365 (1998).
18. L.A. Liotta et al., Cell, 64:327 (1991).
19. L. Smith, et al., Cancer, 107:232 (2006).
20. H. Hung et al., (abstract), American Association for Cancer Research Annual Meeting: Proceedings, Apr. 14-18, 2007; Los Angeles, CA, Philadelphia, PA: AACR, Abstract nr 99 (2007).
21. D.J. Jonker et al., 2007 ASCO Annual Meeting Proceedings Part I, Vol. 25, No. 18S (June 20 Supplement): Abstract # 3559 (2007).
22. M.J. Piccart-Gebhart et al., N. Eng. J. Med., 353:1659 (2006).
23. Early Breast Cancer Trialists' Collaborative Group, Lancet, 351:1451 1 (1998).

### EXAMPLES

### EXAMPLE 1 - IDENTIFICATION OF BIOMARKERS

### Experimental Procedures

Immunohistochemistry (IHC) assays were optimized using the following methods. A positive control tissue or cell line was identified and formalin fixed and paraffin embedded. A specific antibody that recognizes the marker of interest was obtained through commercial sources and tested in the positive tissue. Three different concentrations of the antibody and three different antigen retrieval methods were used. If after the first try, an optimized condition was not found, the dilution of the antibody was expanded or a different antibody was purchased. Below are descriptions of how each antibody was optimized for each of the biomarkers.

### FGF1

First Try: FGF1 antibody was obtained from Santa Cruz (catalogue #sc-1884). It was used in two different titers (1:100; 1:250) with three antigen retrieval conditions (Citra Plus (BioGennex solution; AR10 Biogennex solution; Proteinase K (PK)). Second Try: FGF1 was obtained from Abcam (#ab18963) in one titer (1:100; per vendor recommendation) with three antigen retrieval conditions (Citra Plus; AR10; PK). Third Try: FGF1 antibody was obtained from Abcam (abcam#18963) in two different titers (1:50; 1:75) with PK antigen retrieval.

### Positive Control: Human Colon Tumor PA154777A

Slides were de-paraffin and rehydration in the LBTCA® ST5020 Multistainer in Xylene, 2 times, 5 min each followed by rinsing for 2 times with 100% Ethanol, 2 times of 95% Ethanol, and 2 times in 70% Ethanol, for 3 min/each. This was followed by a washing 3 times in dH₂O. Afterwards, antigen retrieval was done using Proteinase K (PK) for 10 minutes at room temperature (RT). Reagents were set on a BioGennex i6000 autostainer. A pap pen was used to define the area of the tissue to assure the reagents covered only that area. The autostainer was run using the research mode with the following conditions: Blocking with Peroxide Block, 10min, RT followed by power Blocking with 2% Goat Serum, 30min, RT. Then, the slides were incubated with primary antibody (Ab) or rabbit IgG, at RT, for 1hr, followed by HRP-anti-rabbit, for 30min at RT. Chromogen Substrate was added (DAB) and it was let to develop for 5 min. Counterstained with Hematoxyline, 1min.

### FGF2

First Try: FGF2 antibody was obtained from Santa Cruz (catalogue #sc-1390) in two different titers (1:100; 1:250) with three antigen retrieval conditions (Citra Plus; AR10; PK). Second Try: FGF2 antibody was obtained from Upstate (catalogue# 05-118) in one titer (1:100;vendor recommendation) with three antigen retrieval conditions (Citra Plus; AR10; PK). Third Try: FGF2 antibody was obtained from Upstate (05-118) and Santa Cruz (#sc-1390)) in two different titers (1:50-Upstate; 1:50-Santa Cruz) with three antigen retrieval conditions (Citra Plus; AR10; PK). Fourth Try: FGF2 antibody was obtained from Abcam (#ab16828) in two different titers (1:20; 1:40) with three antigen retrieval conditions (Citra Plus; AR10; PK).

### VEGFR-2

First Try: VEGFR-2 antibody was obtained from R&D Systems (#AF357) in two different titers (1:10; 1:100) with three antigen retrieval conditions (Citra Plus; AR10; PK). Second Try: VEGFR-2 antibody was obtained from Upstate (07-158) in one titer (1:100; vendor recommendation) with three antigen retrieval conditions (Citra Plus; AR10; PK). Third Try: VEGFR-2 antibody was obtained from Upstate (07-158) in two different titers (1:200; 1:250) with two antigen retrieval conditions (Citra Plus; AR10). Fourth Try: VEGFR-2 antibody was obtained from Cell Signaling (#2479) in three different titers (1:75; 1:125; 1:250) with three antigen retrieval conditions (Citra Plus; AR10; PK). Fifth Try: VEGFR-2 antibody was obtained from Cell Signaling (#2479) in two different titers (1:35; 1:75) with AR10 antigen retrieval conditions.

### Collagen Type IV

Collagen IV antibody was obtained from Fitzgerald Industries International (RDI) (RDI-PRO10760), which is a rabbit polyclonal IgG. The antigen retrieval we used was Proteinase K for 5 min. A 1 hr incubation was performed at rt with a 1:250 dilution. The Envision kit from Dako was used for detection.

### Clinical Study CA 182-002

Clinical study 182-002 is a phase I dose escalation study to determine the safety, pharmacokinetics, and pharmacodynamics of brivanib in patients with advanced or metastatic solid tumors.

### Immunohistochemistry in Clinical Samples

Optimized protocols were run on the clinical samples. The samples were run in three batches. For each batch, a positive and a negative control were included. Results of IHC staining in the clinical trial CA 182-002:

40 tumor samples were obtained from patients in clinical trial 182-002. These are archived samples obtained prior to therapy. One slide of each patient was stained with hematoxilin and eosin (H&E), which provides the histological staining to determine the type of cancer and the % of tumor present in the sample (See Table 1). Slides were then sent to a pathologist who scored the H&E slides for percentile of tumor present in the sample and the type of tumor present. It was found that seven of the patients' samples did not contain any tumor (see Table 1).

**TABLE 1**

| **H&E Staining of Patient Samples** | | |
|---|---|---|
| Site ID | H&E | % Tumor |
| CA182-002,0001-00305 | fallopian tube benign | |
| CA182-002, 0003-00306 | leiomysarcoma | 50 |
| CA182-002, 0001-00307 | adeno: ampulla af Vater? | 50 |
| CA182-002, 0003-00309 | soft tissue with myxoid arcas | |
| CA182-002, 0003-00310 | adenocarcinoma of liver | 60 |
| CA182,002, 0001-00311 | malignant spindle cell tumor | 80 |
| CA182-002, 0003-00314 | clear cell neoplasm | 65 |
| CA182-002, 0003-00315 | skin with vascular proliferation in upper dermis not overtly malignant | |
| CA182-002, 0003-00317 | clear cell carcinoma | 75 |
| CA 182-002, 0004-00501 | adenocarcinoma in soft tissue | 60 |
| CA182-002, 0006-00502 | adenocarcinoma in soft tissue | 70 |
| CA182-002, 0004-00503 | adenocarcinoma in soft tissue | 70 |
| CAI 82-002, 0009-00504 | papillary renal cell carcinoma | 65 |
| CA182-002, 0003-00505 | clear cell carcinoma | 90 |
| CA182-002,0004-00506 | adenocarcinoma in necrotic debri | 30 |
| CA182-002, 0003 -00507 | fragments of adenocarcinoma with autery artifact | |
| CA182-002, 0006-00509 | adenocarcinoma of the colon | 70 |
| CA182-002, 0004-00510 | liver with metastasis of adenocarcinoma | 30 |
| CA182-002, 0006-00514 | colon with mucinous adenocarcinoma | 70 |
| CA182-002,0004-00516 | colon with invasive adenocarcinoma | 60 |
| CA182-002, 0006-00517 | colon with invasive adenocarcinoma | <10 |
| CA182-002, 0008-00301 | clear cell neoplasm | |
| CA182-002, 0008-00302 | urinary bladder with mucinous adenocarcinoma in wall | 60 |
| CA182-002. 0001-00303 | soft tissue with adenocarcinoma | 60 |
| CA182-002, 0008-00304 | carcinoid | 85 |
| CA 82-002,0008-00308 | adenocarcinoma | 90 |
| CA182-002, 0008-00319 | soft tissue with highly atypical gland cells | |
| CA182-002, 0005-00511 | colon with adenocarcinoma | 50 |
| CA182-002, 0005 -00512 | colon with adenocarcinoma | 30 |
| CA182-002, 0007-00513 | colon with adenocarcinoma. | 40 |
| CA182-002, 0005-00515 | soft tissue with lymph nodes and granulation tissues with giant cells. No differentiate tumor cells | |

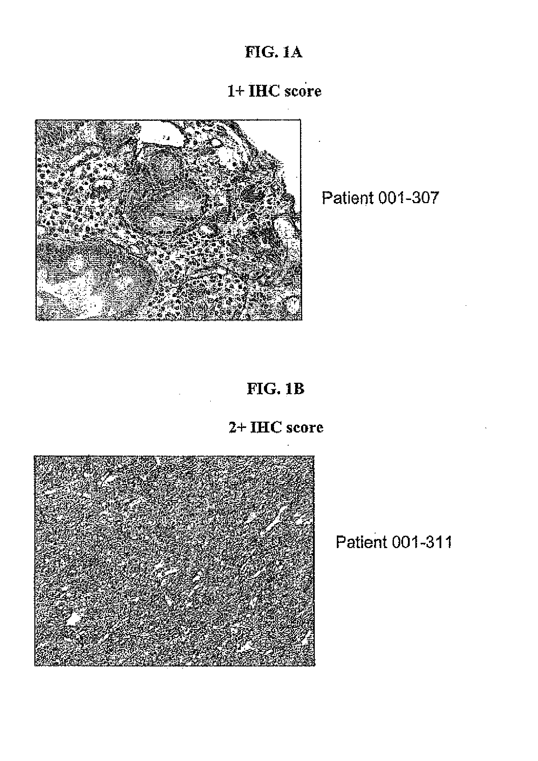

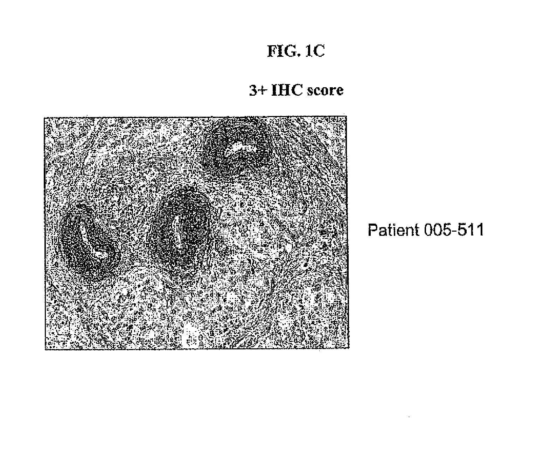

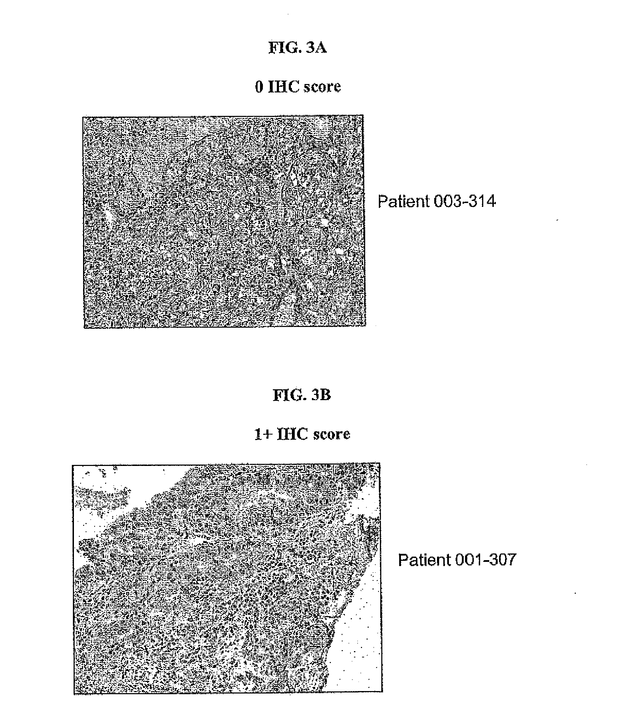

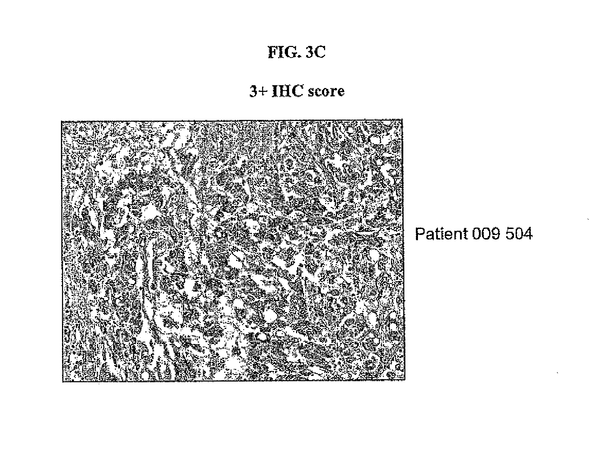

In addition to the H&E experiment, slides of patients were stained using Immunohistochemistry (IHC) for the following markers: FGF1, FGF2, VEGFR-2 and Collagen type IV. Slides were stained with the optimized protocol for each of the markers (see experimental procedure) and sent to a pathologist for evaluation (the pathologist was blinded with regard to patient outcome). The pathologist scored the IHC stained slides for the biomarkers regarding the intensity, localization, and percentage of staining for each of the biomarkers. The scoring that was followed was a standard pathologist scoring using a scale of 1 to 3. The results for that scoring are provided in Table 2 and examples of staining and its scores and shown in FIGS. 1-4.

**TABLE 2**

| **IHC Scoring of Patient Samples from CA 182-002** | | | | |
|---|---|---|---|---|
| Patient ID | Collagen type IV | VEGFR-2 | FGF1 | FGF2 |
| CA182-002, 0001-00305 | 2 | 0 | 0 | 0 |
| CA182-002, 0003-00306 | 1 | 0 | 0 | 0 |
| CA182-002, 0001-00307 | 1 | 0 | 1 | 2 |
| CA182-002, 0003-00309 | 3 | 0 | 0 | 0 |
| CA182-002, 0003-00310 | 1 | 0 | 0 | |
| CA182-002, 0001-00311 | tumor 2 | 0 | 0 | 0 |
| CA182-002, 0003-00314 | 2 | 0 | 0 | 2 |
| CA182-002,0003-00315 | 2 | 0 | 0 | 0 |
| CA182-002, 0003-00317 | 1 | 0 | 0 | 0 |
| CA182-002, 0004-00501 | 3, 3 desmoplasia | 1 | 0 | 0 |
| CA182-002, 0006-00502 | 2 | 0 | 1 | 1 |
| CA182-002, 0004-00503 | 3 | 1 | 0 | 0 |
| CA182-002, 0009-00504 | 3 | 1 | 3 | |
| CA182-002, 0003-00505 | 2 | 2 | 2 | |
| CA182-002, 0004-00506 | 3 | 0 | 0 | 0 |
| CA182-002, 0003-00507 | 2 | 0 | 0 | 2 |
| CA182-002, 0006-00509 | 2 | 0 | 1 | 1 |
| CA 182-002, 0004-00510 | 2 | 0 | 0 | 2 |
| CA182-002, 0006-00514 | 1 | 0 | 0 | 0 |
| CA182-002, 0004-00516 | 3 | 1 | 2 | 3 |
| CA182-002, 0006-00517 | 3 | 0 | 3 | 1 |
| CA182-002, 0008-00301 | 3 | 1 | 0 | 2 |
| CA182-002, 0008-00302 | 3 | 0 | 0 | 1 |
| CA182-002, 0001-00303 | 2 | 0 | 0 | 0 |
| CA182-002, 0008-00304 | 3 | 0 | 0 | 0 |
| CA182-002, 0008-00308 | 3 | 2 | 1 | 1 |
| CA182-002, 0008-00319 | 3 | 2 | 0 | 1 |
| CA182-002, 0005-00511 | 3 | 2 | 0 | 2 |
| CA182-002, 0005-00512 | 3 | 2 | 0 | 2 |
| CA182-002, 0007-00513 | 3 | 0 | 0 | 3 |
| CA182-002, 0005-00515 | 3 | 2 | 0 | 0 |
| CA182-002, 0008-00521 | 0 | 0 | 0 | 40% 2+ cytoplasm |
| CA182-002, 0002-00532 | 0 | 0 | 0 | 0 |
| CA182-002, 0002-00535 | 0 | 0 | <5% 1+ cytoplasm | 90% 2+ cytoplasm |
| CA182-002, 0004-00508 | 0 | <2% 1+ cytoplasm | <2%1+ cytoplasm | 70% 1+ cytoplasm |
| CA182-002, 0004-00520 | 0 | 0 | 0 | 0 |
| CA182-002, 0004-00531 | 0 | 0 | 0 | <10% 1+ cytoplasm |
| CA182-002, 0006-00538 | 0 | 0 | 0 | 0 |
| CA182-002,0006-00539 | 0 | 0 | 0 | <5% 2+ cytoplasm |
| CA182-002, 0009-00534 | 0 | 0 | 0 | 60% 2+ cytoplasm |

Collagen type IV was high in most of the patients, which is what was expected since collagen type IV is overexpressed in other tumors (E. Iochim et al., Eur: J. Cancer, 38(18):2362-2370 (Dec. 2002) and A. Isisag et al., Anal. Quant. Cytol. Histol., 25:263-272 (Oct. 2003)). The high scoring values seen were all in endothelial tissues, the place that this marker is normally present. At the beginning, the staining associated around the endothelial vessels was scored (see FIG. 1C). As the intensity of the staining increased so did the scoring (compare the 1+ in FIG. 1A to the 3+ in FIG. 1C). A couple of patients showed the staining of this marker in the tumor area (see FIG. 1B), which was an interesting observation and it was unclear what this means. Since it was found that all the tumors had high levels of collagen type IV in the biopsies prior to treatment, the staining of this marker in tumors in the last batch of patients was assessed. As a result, the scoring for the last 9 patients was changed and scored only the intensity staining of collagen type IV in tumor patient's s samples (in Table 1 from patient 008-00521 to the end of the table all the patients in the second part of the table).

The staining that was observed with the VEGFR-2 biomarker is starting to distinguish two populations. Based on these results, it seems that high levels of VEGFR-2 are associated with PD patients.

FGF I staining that was observed was interesting in that it looks as it trends with FGF2. All of the FGF1 positive tumors were also positive for FGF2, so they may be connected. This may suggest that both ligands can affect the same pathways.

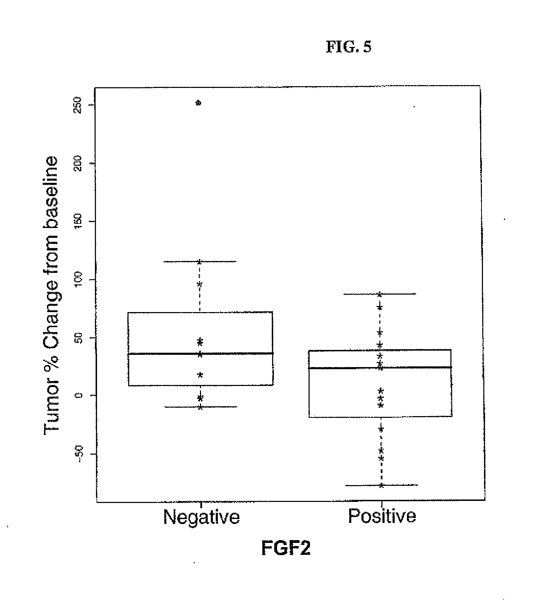

FGF2 IHC staining showed interesting results. The positive expression of the marker seems to be correlated with response and stable disease (SD) greater than four months (SD>4) months in the small number of patients that were studied. If responders were defined as those patients who at the time of the analysis had a partial response (PR) or a stable disease (SD) longer than four months, then the result would be that 4 out of 5 responders are positive for FGF2 IHC staining. Positivity in the IHC assay was defined as any value above 0. Of the rest of the patients defined as non- responders, 12 out of 18 were also positive for FGF2 staining. These results give a sensitivity of 0.8 (95% CI: (0.28, 0.99)) and a specificity of 0.4 (95% CI: (0.19, 0.64)). Thus, giving us a Positive Predictive Value (PPV) of 0.25 with a 95% CI: (0.07, 0.52) and a Negative Predictive Value (NPV) of 0.89 with a 95% CI: (0.52, 1.00). While these correlations are not statistically significant at this point, there is a trend in the direction of patients responding to treatment with brivanib that are positive for FGF2 staining. Another interesting correlation was that there seems to be a correlation of presence of FGF2 and tumor shrinkage. When the percentage tumor shrinkage was plotted from baseline to the time of the analysis, it was found that those tumors that stained with FGF2 tend to have more shrinkage than the negative staining tumors (FIG. 5). Though, due to the small numbers of patients for which medical responses were obtained, the numbers are not statistically significant at this point.

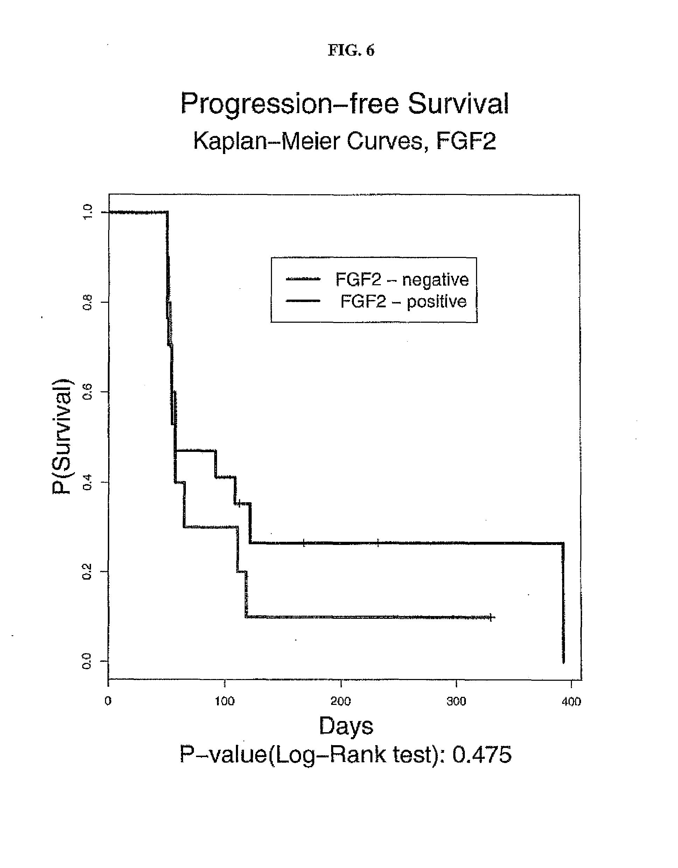

Besides the increase in the tumor shrinkage for the FGF2 positive patients, it was also found that patients treated with brivanib whose tumors were positive for FGF2 tended to have a survival advantage (FIG. 6). Again, while this is not statistically significant (p value 0.475), a separation of the curves in the survival plot is starting to be observed. Thus, indicating that the FGF2 positive tumors tend to live longer.

### EXAMPLE 2 - BRIVANIB MODULATION OF FGF EXPRESSION LEVELS

### Materials and Methods

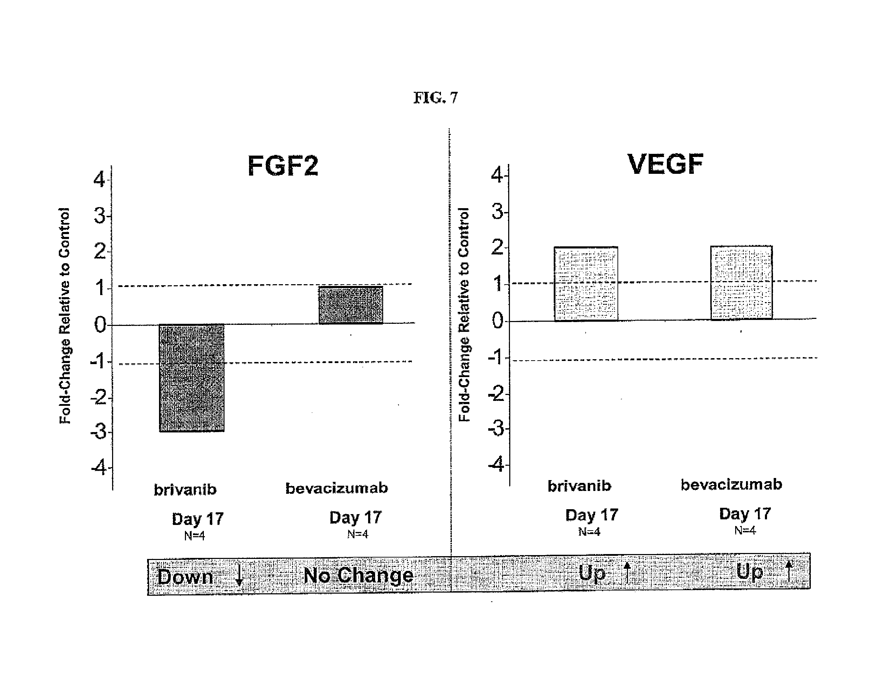

FIG. 7 illustrates results showing that brivanib modulated FGF2 growth factor gene expression levels whereas bevacizumab did not, while both brivanib and bevacizumab modulated VEGF gene expression levels. The results shown are from AFFYMETRIX® human arrays.

Brivanib was dissolved in DMSO at a concentration of 10 mM and stored at -20 °C. Bevacizumab was at a concentration of 25mg/ml and stored at 4 °C.

Human recombinant fibroblast growth factor basic (FGF2) was dissolved in PBS at a concentration of 100 µg/ml and stored at -20°C.

Human recombinant vascular endothelial growth factor (VEGF) was dissolved in PBS at a concentration of 100 µg/ml and stored at -20 °C

### Brivanib Inhibits FGF2-Induced Tumor Cell Proliferation In Vitro

The effects of brivanib (10 nM), sunitinib (10 nM), and bevacizumab (25 mg/ml) AVASTIN® (bevacizumab) on VEGF and FGF2-stimulated cell proliferation was evaluated in the following cancer cell lines: A2870, GEO, L2987, 786-0, A549, A498, Du145, PANC-1, ACHN, HT1197, CALU6, SKOV3, 769-P, T24, HCT166 and UMUC3. Tumor cell inhibition in cell lines stimulated with VEGF (2.5 ng/ml) or FGF2 (2.5 ng/ml) was evaluated with brivanib (0, 0.3, 0.6, 1.25, 2.5 , 5 and 10 µM) and sunitinib (0, 0.3, 0.6, 1.25, 2.5, 5 and 10 µg/ml).

Gene expression profiling was performed on RNA samples from tumor cell lines using AFFYMETRIX® GeneChip System. For this analysis data were available for only 16 (8 sensitive and 8 resistant) cell lines however, 19 cell lines were tested for brivanib sensitivity following FGF2 stimulation.

Cell lines with IC50 ≤ 3µM were considered sensitive, whereas cell lines with IC50 > 3µM were considered resistant.

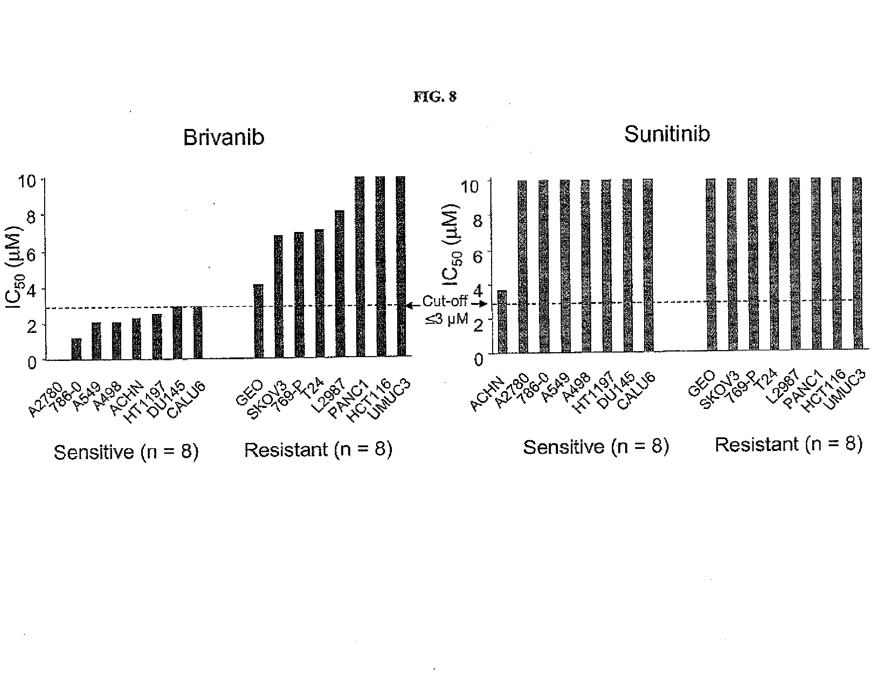

As shown in FIG. 8, brivanib was shown to inhibit FGF2-induced tumor cell proliferation *In vitro* with IC50 values ranging from 0.7 to 10 µM across a panel of 16 cell lines whereas the IC50 values with sunitinib were mostly about 10 µM. Table 3 shows the tissue distribution of the tested cancer cell lines.

**TABLE3**

| ***In Vitro* Brivanib-Sensitivity of Cell Lines Following FGF2 Stimulation** | | |
|---|---|---|
| Cell Lines | Brivanib-sensitive (≤ 3 µM) | Brivanib-resistant (> 3 µm) |
| Renal | 3 | 1 |
| NSCLC | 2 | 1 |
| Bladder | 1 | 2 |
| Ovarian | 1 | 1 |
| Prostate | 1 | 1 |
| Colon | 0 | 3 |
| Pancreatic | 1 | 1 |
| Total | 9 | 10 |

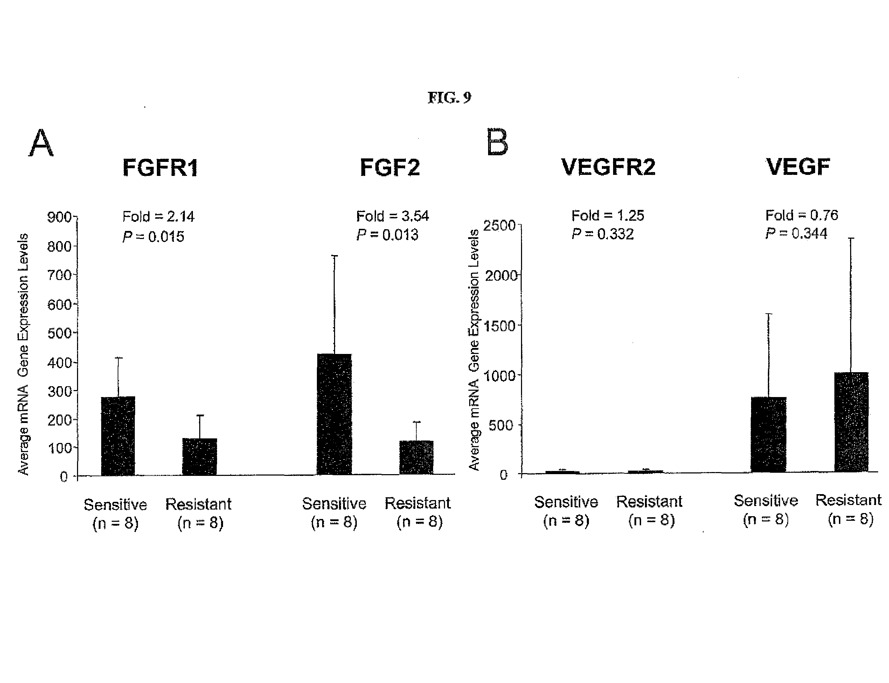

The mRNA expression levels of FGFR1, FGF2, VEGFR2, and VEGF were analyzed in brivanib-sensitive and resistant tumor cell lines. As shown in FIG. 9, FGF2 and FGFR1 RNA expression levels showed a positive correlation with sensitivity to brivanib *in vitro.* Overall higher FGF2 and FGFR1 RNA expression levels were detected in sensitive cell lines. While VEGF was expressed in the range of cell lines, its expression level did not correlate with sensitivity to brivanib *in vitro.* Similarly, VEGFR2 expression was low across all tumor cell lines tested and levels did not correlate with brivanib sensitivity in *vitro.*

### VEGF and FGF2 Stimulating Cell Proliferation

Cell Culture - GEO colon cancer cell line was routinely maintained in RPMI 1640 (Cellgro, Virginia) with 1% fetal bovine serum (Cellgro, Virginia) at 37°C in 5% carbon dioxide/95% air in the presence of 100 units/ml penicillin, 100 µg/ml streptomycin (Cellgro, Virginia).

Cell proliferation was determined by a modified MTS assay with CELLTITER 96® Aqueous One Solution Reagent (Promega, Madison, Wisconsin). A2780 ovarian cancer cells, MIAPAC-2 pancreatic cancer cells, or GEO colon cancer cells were seeded on 96-well flat-bottomed tissue culture plates (Becton Dickinson, San Jose, California) at a concentration of 3 x 10³ cells/well in RPMI 1640 with 1% fetal bovine serum at 37°C in 5% carbon dioxide/95% air in the presence of 100 units/ml penicillin; 100 µg/ml streptomycin and allowed to adhere to the plate overnight. Then the cells were incubated in the presence of each concentration of 0 (control), 0.3, 0.6, 1.25, 2.5, 5, 10 ng/ml of FGF2 or VEGF for another 72 hours at 37°C in a humidified atmosphere of 5% CO₂ in air. After treatment, 20 µl of CELLTITER 96® Aqueous One Solution Reagent was dropped into each well of plates. After 180 minutes incubation, the optical densities (OD) of these samples were directly measured using SPECTRAMAX® plus (Molecular Device, California) and reference wavelength of 490 nm. The OD of control samples was regarded as 100. Each condition was performed with 4 wells and experiment was repeated twice.

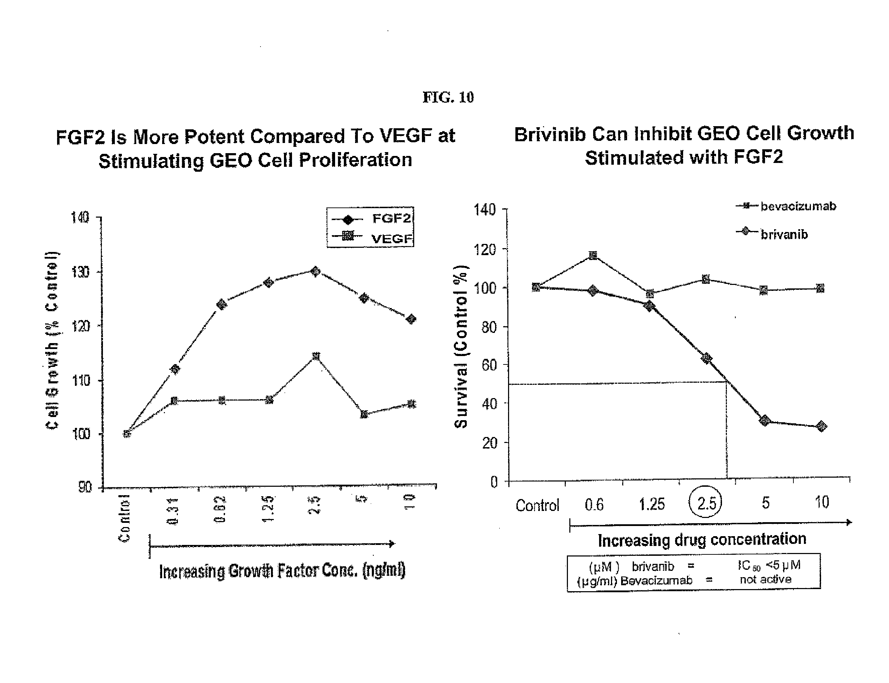

FIG. 10 illustrates the results obtained for FGF2- and VEGF-induced cellular proliferation in GEO colon cancer cells. The proliferative activities of FGF2 and VEGF are shown in terms of cell growth compared to control (%). As shown, brivanib potently inhibited FGF2-induced cellular proliferation in GEO cells, but did not potently inhibit VEGF-induced cellular proliferation.

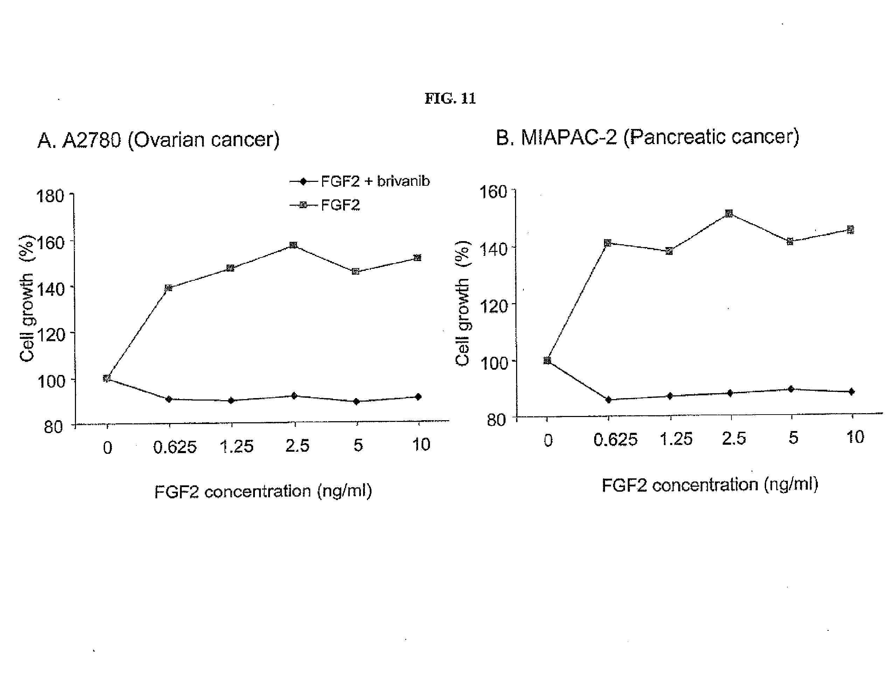

FIG. 11 illustrates the results obtained FGF2-induced cellular proliferation in A2780 ovarian cancer cells and in MIAPAC-2 pancreatic cancer cells. The proliferative activities of FGF2 are shown in terms of cell growth compared to control (%). As shown, brivanib potently inhibited FGF2-induced cellular proliferation in both A2780 ovarian cancer cells and in MIAPAC-2 pancreatic cancer cells. Specifically, Brivanib (IC50 < 1 µM) inhibited FGF2-induced cell growth by > 50% in ovarian cancer cell line A2870, while Brivanib (IC50 < 0.5 µM) inhibited FGF2-induced cell growth by > 50% in the pancreatic cancer cell line MIAPAC-2. Bevacizumab did not show significant sensitivity to FGF2-induced cellular proliferation in ovarian cancer cell line A2870 and in pancreatic cancer cell line MIAPAC-2 (data not shown). Similarly, AVASTIN® did not show significant sensitivity to VFGF-induced cellular proliferation in ovarian cancer cell line A2870 and in pancreatic cancer cell line MIAPAC-2 (data not shown).

### Tumor Cell Inhibition

Cell Culture - GEO colon cancer cell line maintained with 2.5ng/ml VEGF or FGF2 in RPMI 1640 (Cellgro, Virginia) with 1% fetal bovine serum (Cellgro, Virginia) at 37 °C in 5% carbon dioxide 95% air in the presence of 100 units/ml penicillin, 100 µg/ml streptomycin (Cellgro, Virginia) for 14 days.

Cell proliferation was determined by a modified MTS assay with CELLTITER 96® Aqueous One Solution Reagent (Promega, Madison, Wisconsin). GEO cells were seeded on 96-well flat-bottomed tissue culture plates (Becton Dickinson, San Jose, California) at a concentration of 3 × 10³ cells/well in RPMI 1640 with 1% fetal bovine serum at 37 °C in 5% carbon dioxide/95% air in the presence of 100 units/ml penicillin, 100 µg/ml streptomycin and allowed to adhere to the plate overnight. Then, the cells were incubated in the presence of each concentration af 0 (control), 0.3, 0.6, 1.25, 2.5, 5, 10 µM of brivanib or 0.3, 0.6, 1.25, 2.5, 5, 10 µg/ml of bevacizumab. After 30 minutes drug treatment cells were stimulated with concentration of 0 (control), 2.5ng/ml of VEGF or FGF2, for another 72 hours at 37°C in a humidified atmosphere of 5% CO₂ in air. After treatment, 20 µl of CELLTITER 96® Aqueous One Solution Reagent was dropped into each well of plates. After 180 minutes incubation, the optical densities (OD) of these samples were directly measured using SPECTRAMAX® plus (Molecular Device, California) and reference wavelength of 490 nm. The OD of control samples was regarded as 100. Each condition was performed with 4 wells and each experiment was repeated twice. FIG. 10 illustrates the results obtained, wherein brivanib inhibited cancer cell growth directly when FGF2 was used to stimulate proliferation. The anti-proliferative activities of brivanib and bevacizumab are shown in terms of IC₅₀s.

### Tumor Cell Inhibition Over the Time

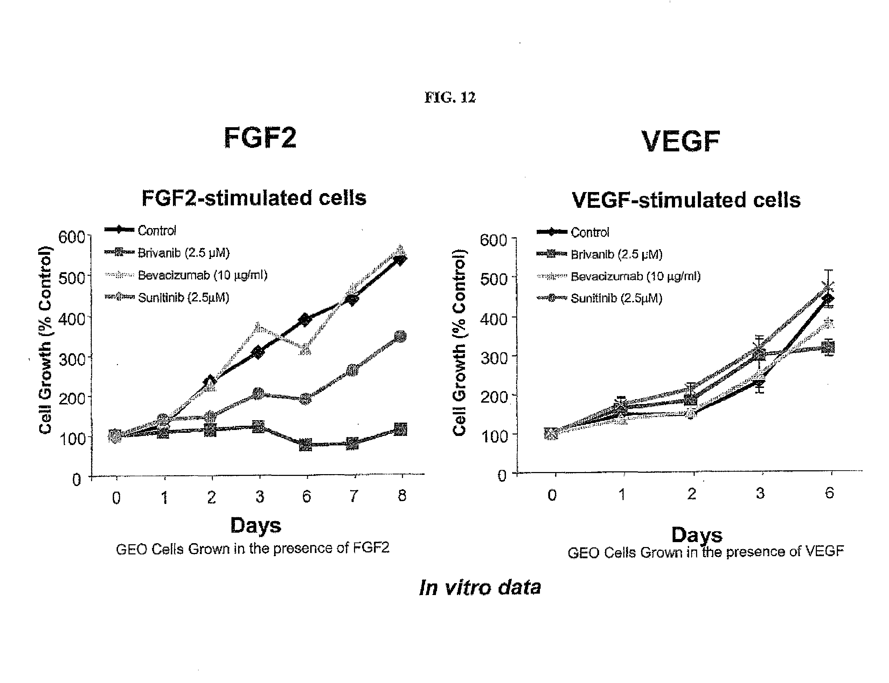

Using the tumor cell inhibition method, cells were treated with concentration of 2.5 µM of brivanib, 10µg/ml of bevacizumab, or 2.5 µM of sunitinib with presence of VEGF or FGF2. Each time point (day 0 (control no treatment)) after treatment with drug day 1, 2, 3, 6, 7, 8, 20 µl of CELLTITER 96® Aqueous One Solution Reagent were dropped into each well of plates. After 180 minutes incubation, the optical densities (OD) of these samples were directly measured using SPECTRAMAX® plus (Molecular Device, California) and reference wavelength of 490 nm. The OD of control samples was regarded as 100. FIG. 12 illustrates the results obtained, wherein brivanib inhibited GEO tumor cell growth stimulated with FGF2 but not VEGF. The anti-proliferative activities of brivanib, sunitnib and bevacizumab are shown in terms of cell growth compared to day 0 control (%) in presence of VEGF and FGF2 condition.

### Effect of Brivanib on Phosphorylation of FGFR1

The phosphylation status of FGFR1, VEGFR2, ERK, and AKT after treatment with 2µm brivanib in the presence of 40ng/ml of FGF2 was analyzed by Western Blots using commercially available anti-phospho primary antibodies directed to each target. Anti-FGFR1, Anti-VEGFR2, Anti-ERK, Anti-AKT, and Anti-alpha tubulin antibodies were also utilized. Blots were incubated with indicated primary antibodies and 1:7500 horseradish peroxidase-conjugated secondary antibodies. All primary antibodies were used at a final concentration of 1 µg/ml. The blots were then visualized with a chemiluminescent detection system.

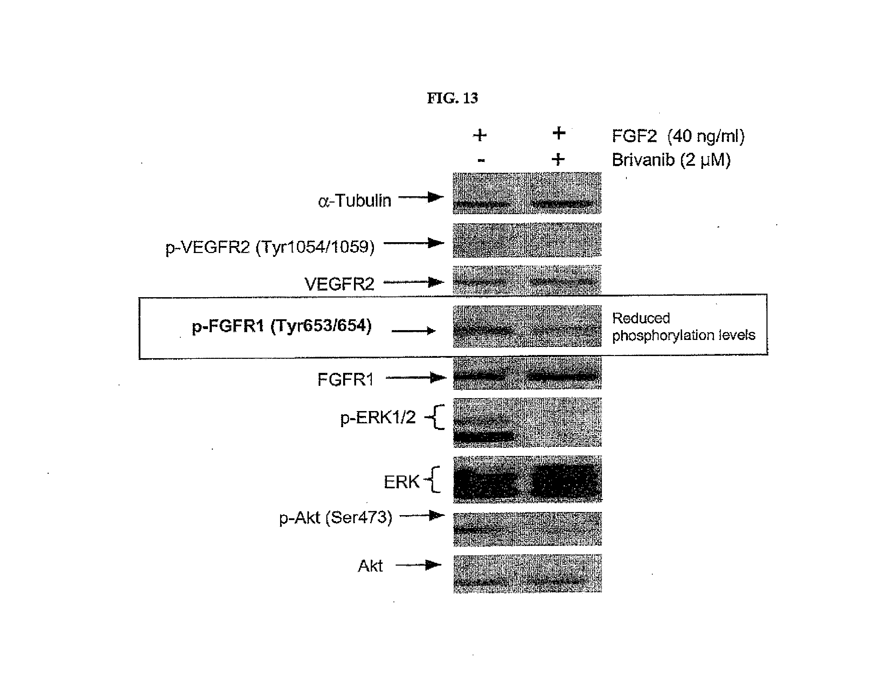

As shown in FIG. 13, brivanib inhibits phosphorylation of FGFR1 following stimulation with FGF2 in the HCC cell line, affecting downstream pathway genes such as pERK and pAKT.

In summary, in *vitro* brivanib inhibited FGF2-induced tumor cell proliferation in 16 cancer cell lines. However, it had no affect on tumor cell proliferation when the cells were grown in the presence of VEGF. FGF2 and FGFR1 RNA expression levels showed a positive correlation with sensitivity to brivanib in *vitro.* Overall higher RNA expression levels were detected in the cell lines sensitive to brivanib. In contrast, VEGF was expressed in all cell lines but levels did not correlate with sensitivity to brivanib *in vitro.* VEGFR2 expression was low across all tumor cell lines tested and the levels did not correlate with brivanib sensitivity in *vitro.* In addition, brivanib inhibited phosphorylation of FGFR1 following stimulation with the FGF2 ligand in *vitro.*

### EXAMPLE 3 - METHOD OF CONFIRMING INVOLVEMENT OF FGF PATHWAY IN BRIVANIB SENSITIVITY

In order to confirm that the FGF pathway is an important predictor of brivanib sensitivity, a gene set enrichment analysis was performed on both sensitive and resistant brivanib cell lines either with or without FGF2-induction, and either in the presence of brivanib or sunitinib. The expression profiles of individual genes within each pathway were analyzed, and the log of the change in expression profile for each condition was measured. Overall, 67 pathways were analyzed. Each gene analyzed for each pathway have not been provided. But, 46 genes from the FGF pathway, 62 genes from the VEGF pathway were analyzed, and a larger generic number of genes consisting of 92 genes for the 1FGF pathway were analyzed using the AMBION® gene set.

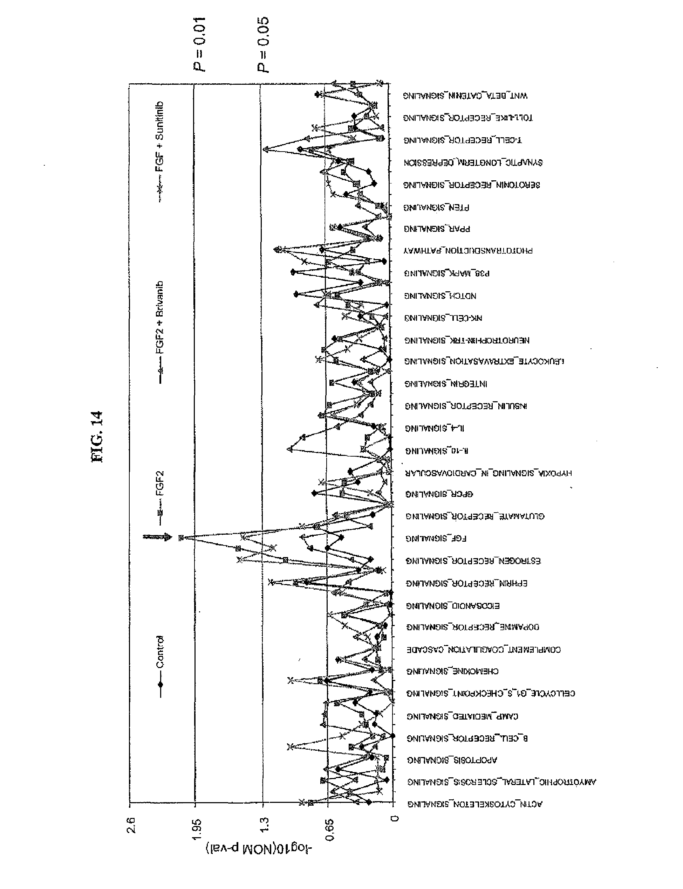

As shown in FIG. 14, the FGF pathway related genes were most significantly induced in brivanib-sensitive cells.

After cells were treated with 1µm brivanib or 1µm sunitinib after FGF2 stimulation, FGF2-induced FGF pathway up-regulation was inhibited only by brivanib, but not sunitinib.

As a result, the presence of the FGF2 ligand is critical for activation of FGFR and the FGF pathway resulting in increased proliferation of tumor cell lines. Based upon the results outlined herein, brivanib is able to inhibit tumor cell proliferation induced by FGF2, and thus increased FGF2 expression is useful as a predictor of sensitivity to brivanib therapy.

### EXAMPLE 4 ― METHOD OF CONFIRMING THE CORRELATION BETWEEN FGF2 TUMOR EXPRESSION WITH TUMOR RESPONSE, PFS, AND CHANGES IN PLASMA PHARMACODYNAMIC (PD) MARKERS FOLLOWING TREATMENT WITH BRIVANIB

### Materials and Methods

### Patients

Patients included in the study were enrolled in a phase 1 clinical trial investigating the safety, pharmacokinetics, pharmacodynamics, and maximal acceptable dose for brivanib alaninate (K. Suzuki et al., Int. J. Urol., 12:152 (2005)). All patients gave informed consent for the clinical trial and for this study, which was approved by the appropriate institutional review boards. Inclusion criteria include ECOG 0-1 (S. Javerzat et al., Trends Mol. Med., 8:483 (2002)), adequate bone marrow and hepatic function, and having tumor tissue block or 20 to 30 unstained slides of the initial biopsy available. Exclusion criteria included prior exposure to vascular endothelial growth factor (VEGF) inhibitors, presence of cardiovascular disease, thromboembolism, or bleeding in the last 6 months. All patients enrolled in the clinical trial had previously failed primary and secondary chemotherapy courses. Sequential cohorts of patients were treated with brivanib alaninate at doses 180 mg to 1000 mg. Patients' response was measured by quantifying tumor size by contrast enhanced tomography (CT).

### Measurement of Tumor Size

Tumor response was assesses using the modified WHO tumor response criteria. Lesions were evaluated by computed tomography (CT). Lesions were bidimensional and had at least one diameter of > 2 cm on standard CT and > 1 cm on spiral CT. For bidimensional measurements, the second diameter was perpendicular to the longest diameter. The area of lesion was calculated by multiplying the longest diameter by the greatest perpendicular diameter.

### FGF-2 Immunohistochemistry

Tumor blocks were sectioned at 4 µm. Slides were de-paraffined and rehydrated in the LEICA® ST5020 Multistainer. Antigen retrieval was carried out by heating slides with Citra Plus Antigen retrieval solution (BioGenex, Cat # HK080-9K) in a BIOGENEX® EZ-Retriever Microwave at 95°C for 15 minutes. The slides were then cooled at room temp for 5 minutes, and rinsed with distilled water for three times (~1 minute each). Slides were immunostained in the i6000 Automated Staining System (BioGennex) using the following conditions: blocking with peroxide block (DAKO, Cat# K4007) for 5 minutes, followed by 5% goat serum for 30 mins, primary antibodies rabbit anti-fibroblast growth factor-2 (FGF-2) (Abcam Cat# #116828) 1:25 or rabbit immunoglobulin G (Rabbit IgG, LabVision, Cat# NC-100-P) 5 µg/ml were added and incubated for 1 hour, followed by Peroxidase Anti-Rabbit IgG (H+L) secondary antibody (Vector, Cat# PI-1000) at 1:200 dilution for 45 minutes. Chromogen substrate DAB (DAKO, Cat# K4007) was added and incubated for 5 minutes. Slides were counterstained with hematoxyline (DAKO cat# S3309) for 1 minute. The stained slides were dehydrated and coverslipped in the LEICA® ST5020 Multistainer and assessed by a board-certified pathologist, without knowledge of patients' medical status.

### Statistical Analysis

To compare response rates between the FGF-2(+) and FGF-2(-) groups, a Fisher's exact test was used. To test for differences of percent changes from baseline in tumor between FGF-2(+) and FGF-2(-) groups, a non-parametric Wilcoxon rank-sum test was used (since the assumptions on the marginal distributions for the t-statistic were slightly violated for percent change from baseline, and small sample sizes could increase the test's vulnerability to assumption violations). The same method was applied to test for the difference in percent change from baseline of CIV at Day 8 and Day 26 between the two groups. In addition, a Rank-based ANCOVA was used to investigate the effect of the FGF-2 status on the post-baseline tumor volume, adjusted for the baseline tumor volume. All analyses were performed using R (Y. Shing et al., Science., 223:1296 (1984)).

### Results

43 evaluable patients with available archival tumor tissue were analyzed for correlations between FGF2 status (+ or -) and brivanib treatment dose with tumor response, PFS, and changes in PD plasma markers. 19 pts were FGF2(-) and 24 pts were FGF2(+). Low (<600 mg) and high (≥600 mg) doses of brivanib were given to 17 and 26 patients, respectively. The patients had a variety of tumor types: 28 (65%) had colorectal cancer (CRC), 5 (12%) had renal cell carcinoma (RCC), and the remaining 10 had a range of tumors, including bladder, pancreatic, ovarian, neuroendocrine, and muscle. Biopsy samples, obtained prior to treatment, were sectioned and stained for FGF-2 expression. According to the results, tumors were divided into FGF-2(-) or FGF-2(+) depending on the staining observed. All groups of patients, except those receiving high-dose brivanib that were FGF2(+) patients, had an increased average change in mean tumor size.

FGF status appeared to predict a patient's response to brivanib. Patients were classified as having progressive disease, stable disease, or a partial response to brivanib treatment according to standard WHO criteria from computed tomography scans. After 56 days of treatment, significantly more patients with FGF-2(+) tumors (62.5% [15 out of 24]) had a partial response or stable disease compared with patients with FGF-2⁻ tumors (26.3% [5 out of 19]; *P* = 0.03). In addition, fewer patients with FGF-2(+) tumors had progressive disease (9 out of 24) than those with FGF-2(-) tumors (14 out of 19). If positive FGF-2 status is used to predict response, the positive predictive value (PPV) is 63% (95% CI is 0.41-0.81) and the negative predictive value (NPV) is 74% (95% CI is 0.49-0.91), indicating that a patient that is positive for FGF2 has 63% chance of responding to treatment with brivaninb alaninate, while if it is negative a 74% chance of not responding.

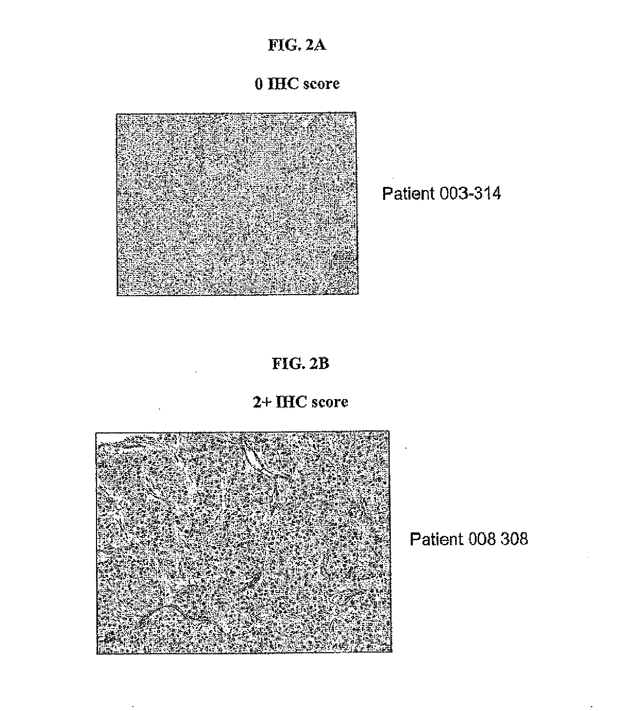

Figure 2 shows the largest percent change in tumor size from baseline in patients with FGF-2(+) tumors compared with patients with FGF-2(-) tumors after two cycles of treatment (approximately 56 days). There was a median increase of 36% (interquartile range [IQR] = 44%) in tumor size in patients with FGF-2(-) tumors compared with only a 1% increase (IQR = 42%, P = 0.007) in patients with FGF-2(+) tumors. Therefore, FGF-2(+) tumors have a clear and significant association with a decrease in tumor growth in brivanib alaninate- treated patients compared with FGF-2⁻ tumors. Differences in tumor responses, PR, and SD ≥8 wks for FGF2(+) and FGF2(-) patients are shown in Table 4.

Median PFS was also longer in FGF2(+) patients than FGF2(-) patients (see Table 4).

In conclusion, whereas FGF overexpression is typically correlated with poor prognosis, FGF2(+) tumor expression was associated with a trend for improved tumor response, PFS, and changes in plasma PD markers following treatment with brivanib compared with patients whose tumors did not express FGF2, and therefore FGF-2 is a biomarker that predicts the responsiveness to brivanib alaninate treatment. Indeed, treatment with brivanib alaninate resulted in a significant decrease in tumor size in patients with FGF-2(+) tumors compared with patients with FGF-2(-) tumors. Furthermore, significantly more patients with FGF-2(+) tumors had better disease control rate (partial response and stable disease) compared with patients with FGF-2(-) tumors.

Although the patients in this study were heavily pre-treated with other therapies, the fact that some were able to achieve a stable disease or even partial improvement demonstrates the potent activity of brivanib alaninate in those patients with FGF-2(+) tumors. In these patients, brivanib alaninate treatment reduced tumor growth by three-fold compared with patients with FGF-2(-) tumors. Therefore, the presence of FGF-2 may be a strong predictive indicator for using therapies that target both the FGF and VEGF pathways, such as brivanib alaninate. These studies also propose the hypothesis that pre-selecting patients based on their FGF-2 status may improve the chances of a positive therapeutic response.

Therefore, FGF-2 status has been shown to be useful in predicting therapeutic responses to brivanib alaninate, a tyrosine kinase inhibitor with dual anti-FGFR1 and anti-VEGR2 activity, for the first time. FGF-2 expression in a variety of tumor types suggests that predicting patient response to therapy across tumor types based on biomarker expression is an achievable goal. In the future, screening patients for FGF-2 status across tumor types may become common practice for considering FGFR- or VEGFR-targeted therapies, alone or in combination with CIV monitoring of patient responses.

**TABLE 4**

| **Correlation Between FGF2 Expression Status and Brivanib Patient Response** | | | | | | | | | |
|---|---|---|---|---|---|---|---|---|---|
| | Mean change in tumor size (%) (~8 wks) | | Median PFS (days) | | COL IV change from baseline (%) (Day 26) (n = 42) | sVEGFR2 change from baseline (%) (Day 26) (n-39) | PR | SD≥8 wks | PD |
| | Low (<600 mg) (n = 17) | High (≥600 mg) (n = 26) | Low (<600 mg) (n = 17) | High (≥600 mg) (n=26) | | | | | |
| FGF2-(n = 19) | 71 | 24 | 54 | 56 | -16 | -15 | 0 | 5 | 14 |
| FGF2+ (n = 24) | 28 | 0.9 | 71 | 107 | -30 | -23 | 2 | 13 | 9 |
| *P* value | 0.03* | | 0.075^{†} | | 0.029* | 0.138* | | | |

| | | | | | | | | | |
|---|---|---|---|---|---|---|---|---|---|
| *Wilcoxon rank-sum test (combined low and high dose assessing FGF2- vs FGF2+). ^{†}Log-rank test (combined low and high dose assessing FGF2- vs FGF2+). | | | | | | | | | |

### EXAMPLE 5 - METHOD OF CONFIRMING THE CORRELATION BETWEEN COLLAGEN TYPE IV EXPRESSION AND PHARMACODYNAMIC RESPONSE AND PREDICTION ALONE AND IN CONJUNCTION WITH FGF2 TUMOR EXPRESSION WITH BRIVANIB

### Materials and Methods

Methods were performed as outlined in Example 4, in addition to the methods outlined *infra.*

### Collagen IV ELISA

Plasma collagen IV (CIV) was measured using an enzyme-linked immunosorbent assay (ELISA) kit obtained from Biotrin International, Ltd. (Dublin, Ireland). The kit was used according to the manufacturer's suggested instructions. Samples included the following visits: baseline (prior to brivanib alaninate treatment) and days 8 and 26, Briefly, quality control samples (QCs) were prepared by spiking a human recombinant collagen IV protein (Southern Biotech, Birmingham, AL) into EDTA plasma (Bioreclamation, Hicksville, NY). Per the manufacturer's kit insert and instructions, the kit standards, QCs and EDTA plasma patient samples were diluted with conjugate (anti-collagen IV mouse Fab' conjugated to horseradish peroxidase, containing 30 mg/l Proclin 300 as a preservative). Diluted samples were incubated for 30 minutes in wells coated with a second anti-collagen IV antibody, and then washed 3 times with kit wash buffer (phosphate buffer with Tween 20 and proclin 300 as a preservative. Ready to use TMB substrate was added and incubated for 30 minutes. The reaction was stopped by the addition of 1M H₂SO₄. The absorbance at 450 nm using 630 nm as a reference was measured using a SPECTRAMAX® plate reader (Molecular Devices, Sunnyvale, CA). Softmax Pro 4.8 software was used to generate a 4-parameter logistic (4-PL) curve fit using the CIV kit standards.

### Results

Changes in CIV levels were also evaluated by ELISA in patients. Patients who were receiving brivanib alaninate at doses of 320 mg and 800 mg on days 8 and 26, regardless of FGF-2 status, were assessed. There was a significant reduction in median CIV levels in patients receiving brivanib alaninate 800 mg compared with brivanib alaninate 320 mg after 8 days (24% vs 2.6%; P < 0.05) and 26 days (31% vs 2.3%;P<0.01).

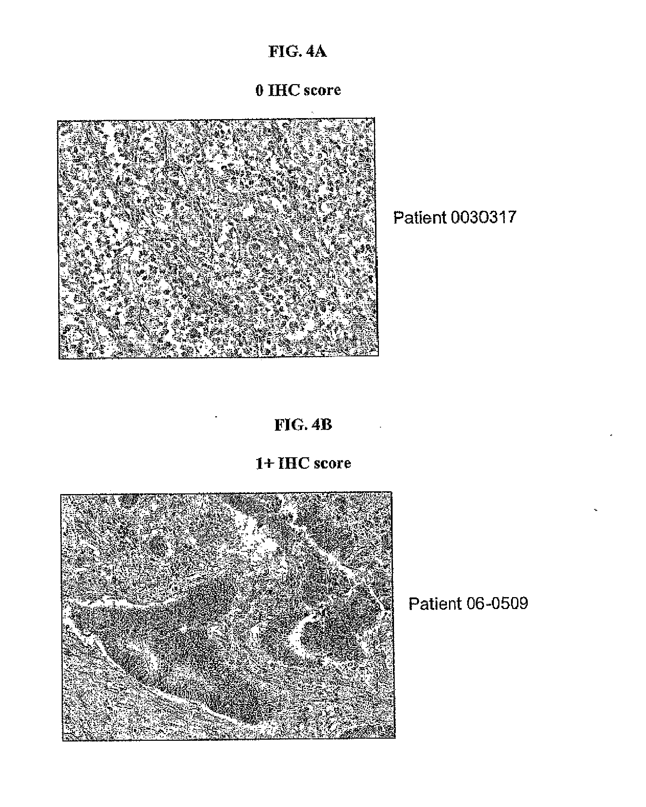

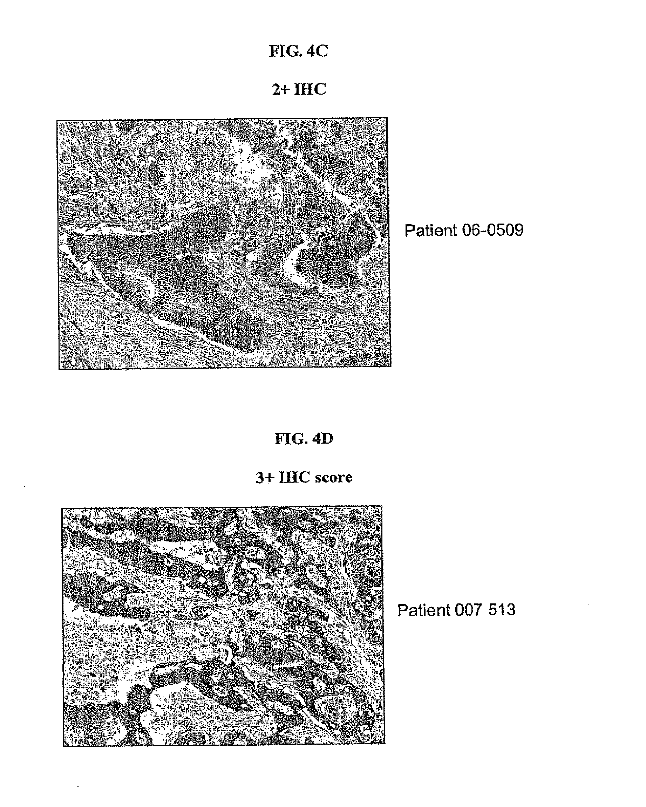

Percent changes in CIV levels were measured in patients with FGF-2(+) and FGF-2(-) tumors (Fig. 4). After 8 days of brivanib alaninate treatment, CIV levels decreased from baseline levels in patients with both FGF-2(+) and FGF-2(-) tumors although the median decrease was 3.3-fold greater for those with FGF-2(+) tumors (16.1% [IQR = 31.0%]) than FGF2(-) tumors (4.9% [IQR = 20.0%]; P = 0.049) (Fig. 4A). The results are even more striking after 26 days. The median reduction from baseline in CIV levels in patients with FGF-2(+) tumors was 30.7% compared with 4.3% (P = 0.019) in those with FGF-2(-) tumors, which represents an approximate seven-fold greater response (Fig. 4B). Interestingly, the CIV changes also correlated with tumor shrinkage (data not shown). These results demonstrate a significantly greater decline in collagen type IV in FGF-2⁺ patients compared with FGF-2⁻ patients.

This is the first demonstration that CIV measurement can be used for an easy and rapid method to assess patient response to this therapy. For the first time, CIV has been demonstrated to be a pharmacodynamic biomarker indicative of patient response to therapy, in general, and for brivanib, specifically. The correlation of declining CIV with the patients' samples that are FGF-2(+), with tumor shrinkage and the correlation of FGF-2(+) with better outcome to treatment with brivanib alaninate further demonstrates the utility of using this biomarker.

For some tumor types, screening for predictive biomarkers is becoming standard clinical practice. For example, overexpression of the human epidermal growth factor receptor-2 (*HER2*)*,* which occurs in 10-34% of invasive breast cancers, can predict a therapeutic response to trastuzumab therapy (M.J. Piccart-Gebhart et al., N. Eng. J. Med., 353:1659 (2006)). Additionally, estrogen-positive status predicts a therapeutic response to anti-estrogen therapies in patients with metastatic breast cancer (Early Breast Cancer Trialists' Collaborative Group, Lancet, 351:1451 (1998)).

Thus, screening patients for FGF-2 status across tumor types may become common practice for considering FGFR- or VEGFR-targeted therapies, either alone, or in conjunction with CIV monitoring of patient responses.

### EXAMPLE 6 - PRODUCTION OF ANTIBODIES AGAINST THE BIOMARKERS

Antibodies against the biomarkers can be prepared by a variety of methods. For example, cells expressing an biomarker polypeptide can be administered to an animal to induce the production of sera containing polyclonal antibodies directed to the expressed polypeptides. In one aspect, the biomarker protein is prepared and isolated or otherwise purified to render it substantially free of natural contaminants, using techniques commonly practiced in the art. Such a preparation is then introduced into an animal in order to produce polyclonal antisera of greater specific activity for the expressed and isolated polypeptide.

In one aspect, the antibodies of the invention are monoclonal antibodies (or protein binding fragments thereof). Cells expressing the biomarker polypeptide can be cultured in any suitable tissue culture medium, however, it is preferable to culture cells in Earle's modified Eagle's medium supplemented to contain 10% fetal bovine serum (inactivated at about 56 °C), and supplemented to contain about 10 g/l nonessential amino acids, about 1,00 U/ml penicillin, and about 100 µg/ml streptomycin.

The splenocytes of immunized (and boosted) mice can be extracted and fused with a suitable myeloma cell line. Any suitable myeloma cell line can be employed in accordance with the invention, however, it is preferable to employ the parent myeloma cell line (SP2/0), available from the ATCC®. After fusion, the resulting hybridoma cells are selectively maintained in HAT medium, and then cloned by limiting dilution as described by Wands et al. (Gastroenterology, 80:225-232 (1981)). The hybridoma cells obtained through such a selection are then assayed to identify those cell clones that secrete antibodies capable of binding to the polypeptide immunogen, or a portion thereof.

Alternatively, additional antibodies capable of binding to the biomarker polypeptide can be produced in a two-step procedure using anti-idiotypic antibodies. Such a method makes use of the fact that antibodies are themselves antigens and, therefore, it is possible to obtain an antibody that binds to a second antibody. In accordance with this method, protein specific antibodies can be used to immunize an animal, preferably a mouse. The splenocytes of such an immunized animal are then used to produce hybridoma cells, and the hybridoma cells are screened to identify clones that produce an antibody whose ability to bind to the protein-specific antibody can be blocked by the polypeptide. Such antibodies comprise anti-idiotypic antibodies to the protein-specific antibody and can be used to immunize an animal to induce the formation of further protein-specific antibodies.

Alternatively, VEGFR2, FGF2, and CIV antibodies are known in the art and are commercially available.

For FGF2, any of the following antibodies may be used in the assays outlined herein, where applicable: FGF-2 (147): sc-79 (Santa Cruz Biotechnology); FGF-2 (3H2133): sc-71105 (Santa Cruz Biotechnology); FGF-2 (500-M38): sc-65350 (Santa Cruz Biotechnology); FGF-2 (AS24): sc-52765 (Santa Cruz Biotechnology); FGF-2 (AS25): sc-52766 (Santa Cruz Biotechnology); FGF-2 (A-7): sc-74413 (Santa Cruz Biotechnology); FGF-2 (C-18): sc-1360 (Santa Cruz Biotechnology); FGF-2 (C-2): sc-74412 (Santa Cruz Biotechnology); FGF-2 (F-343): sc-52845 (Santa Cruz Biotechnology); FGF-2 (F-474): sc-52846 (Santa Cruz Biotechnology); FGF-2 (F-343): sc-52845 (Santa Cruz Biotechnology); FGF-2 (F-474): sc-52846 (Santa Cruz Biotechnology); FGF-2 (F-74): sc-52847 (Santa Cruz Biotechnology); FGF-2 (FB-8): sc-53119 (Santa Cruz Biotechnology); FGF-2 (H-131): sc-7911 (Santa Cruz Biotechnology); FGF-2 (MC-GF1): sc-57125 (Santa Cruz Biotechnology); or FGF-2 (N-19): sc-1390 (Santa Cruz Biotechnology).

For Collagen IV, any of the following antibodies may be used in the assays outlined herein, where applicable: Collagen Type IV (C IV 22): sc-59813 (Santa Cruz Biotechnology); Collagen Type IV (C-19): sc-9302 (Santa Cruz Biotechnology); Collagen Type IV (COL-94): sc-59814 (Santa Cruz Biotechnology); Collagen Type IV (G-20): sc-9301 (Santa Cruz Biotechnology); Collagen Type IV (H-234): sc-11360 (Santa Cruz Biotechnology); Collagen Type IV (N-16): sc-18177 (Santa Cruz Biotechnology); Collagen Type IV (SPM131): sc-56517 (Santa Cruz Biotechnology); or Collagen Type IV (T-15): sc-18178.

For VEGFR2, any of the following antibodies may be used in the assays outlined herein, where applicable, in addition to any antibodies directed to VEGFR2 outlined herein: Flk-1 (A-3): sic-6251 (Santa Cruz Biotechnology); Flk-1 (C-1158): sc-504 (Santa Cruz Biotechnology); Flk-1 (EIC): sc-57135 (Santa Cruz Biotechnology); Flk-1 (EWC): sc-57136 (Santa Cruz Biotechnology); Flk-1 (KDR-2): sc-57134 (Santa Cruz Biotechnology); Flk-1 (N-931): sc-505 (Santa Cruz Biotechnology); Flk-1 (Q-20): sc-19530 (Santa Cruz Biotechnology); Flk-1 (S-20): sc-48161 (Santa Cruz Biotechnology); Flk-1 (xx13): sc-74001 (Santa Cruz Biotechnology); Flk-1 (Y-23): sc-74002 (Santa Cruz Biotechnology); p-Flk-1 (Tyr 951): sc-16628 (Santa Cruz Biotechnology); or p-Flk-1 (Tyr 996)-R: sc-16629-R (Santa Cruz Biotechnology).

### EXAMPLE 7 - IMMUNOFLUORESCENCE ASSAYS

The following immunofluorescence protocol may be used, for example, to verify FGF2, VEGFR-2, or Collagen IV biomarker protein expression on cells or, for example, to check for the presence of one or more antibodies that bind FGF2, VEGFR-2, or Collagen IV biomarkers expressed on the surface of cells. Briefly, LAB-TEK® II chamber slides are coated overnight at 4 °C with 10 micrograms/milliliter (µg/ml) of bovine collagen Type II in DPBS containing calcium and magnesium (DPBS++). The slides are then washed twice with cold DPBS++ and seeded with 8000 CHO-CCR5 or CHO pC4 transfected cells in a total volume of 125 µl and incubated at 37 °C in the presence of 95% oxygen / 5% carbon dioxide.

The culture medium is gently removed by aspiration and the adherent cells are washed twice with DPBS++ at ambient temperature. The slides are blocked with DPBS++ containing 0.2% BSA (blocker) at 0-4 °C for one hour. The blocking solution is gently removed by aspiration, and 125 µl of antibody containing solution (an antibody containing solution may be, for example, a hybridoma culture supernatant which is usually used undiluted, or serum/plasma which is usually diluted, e.g., a dilution of about 1/100 dilution). The slides are incubated for 1 hour at 0-4 °C. Antibody solutions are then gently removed by aspiration and the cells are washed five times with 400 µl of ice cold blocking solution. Next, 125 µl of 1 µg/ml rhodamine labeled secondary antibody (e.g., anti-human IgG) in blocker solution is added to the cells. Again, cells are incubated for 1 hour at 0-4 °C.

The secondary antibody solution is then gently removed by aspiration and the cells are washed three times with 400 µl of ice cold blocking solution, and five times with cold DPBS++. The cells are then fixed with 125 µl of 3.7% formaldehyde in DPBS++ for 15 minutes at ambient temperature. Thereafter, the cells are washed five times with 400 µl of DPBS++ at ambient temperature. Finally, the cells are mounted in 50% aqueous glycerol and viewed in a fluorescence microscope using rhodamine filters.

### EXAMPLE 8 - METHOD OF MEASURING THE LEVEL OF FGF2 EXPRESSION USING IMMUNOHISTOCHEMISTRY ASSAYS

As outlined herein, FGF2(+) cells are sensitive to FGF2 inhibitors and dual FGF2/VEGFR2 inhibitors. Methods of ascertaining whether a cell is FGF2(+) are well known in the art. One method is to use immunohistochemistry (IHC) assays to determine whether cells express FGF2. Briefly, a number of IHC methods are known in the art and can be used to detect the expression level of FGF2 or to determine whether cells are FGF2(+).

Generally, IHC may be followed essentially as described herein. Briefly:

### Preparation of Slides

### A. Primary Cell Lines and Cell Lines

Grow cultured primary cells isolated from patient tissues, or cell lines, on sterile glass cover slips or slides overnight at 37°C. Wash briefly with PBS. Fix as desired. Fix cells by incubating them for 10 minutes with 10% formalin in PBS (keep wet); 5 minutes with ice cold methanol, allow to air dry; and 5 minutes with ice cold acetone, allow to air dry. Then wash in PBS.

### B. Frozen Sections

Snap frozen fresh tissues in liquid nitrogen or isopentane pre-cooled in liquid nitrogen, embedded in OCT compound in cryomolds. Store frozen blocks at - 80 °C. Cut 4-8 µm thick cryostat sections and mount on SUPERFROST PLUS® slides or gelatin coated slides. Store slides at - 80 °C until needed.

Before staining, warm slides at room temperature for 30 minutes and fix in ice cold acetone for 5 minutes. Air dry for 30 minutes. Wash in PBS.

### C. Paraffin Sections

Deparaffinize sections in xylene, 2x5min, Hydrate with 100% ethanol, 2x3min. Hydrate with 95% ethanol, 1min. Rinse in distilled water. Follow procedure for pretreatment as required.

### Pretreatments of Tissue Sections

If the samples were either formalin-fixed or paraffin embedded, antigenic determinants can be exposed by epitope unmasking, enzymatic digestion or saponin, etc.

### Procedure

Rinse sections in PBS-Tween 20 for 2x2min.

Follow the steps below in sequence.

**Serum Blocking:** incubate sections with normal serum block - species same as secondary antibody, for 30 minutes to block non-specific binding of immunoglobulin. Note: since this protocol uses avidin-biotin detection system, avidin/biotin block may be needed based on tissue type. If you do, the avidin/biotin block should be done after normal serum block.

**Primary Antibody:** incubate sections with primary antibody at appropriate dilution in primary antibody dilution buffer for 1 hour at room temperature or overnight at 4°C. Rinse in PBS-Tween 20

**Peroxidase Blocking:** incubate sections in peroxidase blocking solution for 10 minutes at room temperature. Rinse in PBS-Tween 20.

**Secondary Antibody:** incubate sections with biotinylated secondary antibody at appropriate dilution in PBS for 30 minutes at room temperature.

Rinse in PBS-Tween 20 for 3x2min.

**Detection:** incubate sections in streptavidin-HRP (1:500, Vector Labs) in PBS for 30 minutes at room temperature.

Rinse in TBS for 3x2min.

**Chromogen/Substrate:** incubate sections in DAB solution for 1-3 minutes.

Rinse in PBS-Tween 20 2x2min.

**Counterstain** if desired.

Rinse in distilled water.

Dehydrate through 95% ethanol for 2 min, 100% ethanol for 2x3min.

Clear in xylene for 2x5min.

Coverslip with mounting medium.

Other methods of IHC are known in the art and include, using alkaline phosphatase based detection, secondary antibody detection of primary antibody binding, avidin-biotin, immunofluorescence, catalyzed signal amplification, etc.

In addition, some IHC methods used in the art are optimized for clinical diagnosis. One such method is described as PHARMDX® IHC. Briefly,

### (I) Specimen Preparation

Biopsy specimens must be handled to preserve the tissue for IHC staining. Standard methods of tissue processing should be used for all specimens (see D.C. Sheehan, et al., Theory and Practice of Histotechnology, The C. V. Mosby Co., St. Louis (1980)).

### (i) Paraffin-Embedded Sections

Formalin-fixed and paraffin-embedded tissues are suitable for use. Alternative fixatives have not been validated and may give erroneous results. Specimens from the biopsy should be blocked into a thickness of 3 or 4 mm and fixed for the time period appropriate for the fixative. The tissues are then dehydrated and cleared in a series of alcohols and xylene, followed by infiltration by melted paraffin. The paraffin temperature should not exceed 60°C. Properly fixed and embedded tissue blocks expressing the c-kit protein will keep indefinitely prior to sectioning and slide mounting if stored in a cool place (15-25°C).

Tissue specimens should be cut into sections of 3-5 µm. After sectioning, tissues should be mounted on Fisher's SUPERFROST PLUS®, DakoCytomation's Silanized (code S3003), charged slides or poly-L-lysine coated slides and placed in drying racks. The slide racks should be pounded on an absorbent towel to remove water trapped under paraffin and on glass and then dried at room temperature for one hour. The rack of slides should then be placed in a 56-60°C incubator for one hour. Any excess water remaining on slides after removal from the incubator should be removed by pounding slides on towels and drying for one additional hour in the incubator. After removal from the incubator, slides should be held at room temperature until cool and paraffin has hardened. To preserve antigenicity, tissue sections, mounted on slides, should be stained within 2 months of sectioning when held at room temperature (20-25°C). Consult the DakoCytomation Handbook: "Immunochemical Staining Methods" or References 14 and 15 for further details on specimen preparation. The use of decalcified tissues has not been validated and is not recommended. The slides required for FGF2 evaluation and verification of tumor presence should be prepared at the same time. A minimum of 5 slides should be prepared, 1 slide for tumor presence, 2 slides for c-kit protein evaluation, and 2 slides for back-up.

### (II) Reagent Preparation

The following reagents must be prepared prior to staining:

### Target Retrieval Solution (Code S1699)

Prepare a sufficient quantity of Target Retrieval Solution by diluting Target Retrieval Solution 10x 1:10 using distilled or deionized water (reagent-quality water) for the wash steps. Discard Target Retrieval Solution after use.

**Note:** When using DakoCytomation's Target Retrieval Solution (Code S1700) no dilution is necessary.

### Wash Buffer Solution (Code S3006)

Prepare a sufficient quantity of Wash Buffer by diluting Wash Buffer 10x, 1:10 using distilled or deionized water (reagent-quality water) for the wash steps. Store unused solution at 2-8°C for no more that 7 days. Discard buffer if cloudy in appearance.

### Substrate-Chromogen Solution (DAB+) (Code 3467 or K3468)

This solution should be mixed thoroughly prior to use. Any precipitate developing in the solution does not affect staining quality. To prepare DAB+ Substrate-Chromogen Solution, add 1 drop of Liquid DAB+ Chromogen to one mL of DAB+ Substrate Buffer and mix. Discard any unused solution. Stability of prepared DAB+ is approximately 5 days when stored at 2-8°C.

**Important Note:** The color of the Liquid DAB+ Chromogen in the bottle may vary from clear to light lavender brown. Dilute per the guidelines above. Addition of excess Liquid DAB+ Chromogen to the DAB+ Substrate Buffer will result in deterioration of the positive signal.

### Mounting Medium

Non-aqueous, permanent mounting media such as DakoCytomation Ultramount (code S1964) is recommended. Aqueous mounting media such as DakoCytomation Faramount Mounting Medium, Ready-to-use (code S3025) or DakoCytomation GLYCERGEL® Mounting Medium (code C0563) is also suitable. Liquefy GLYCERGEL® by warming to approximately 40(±5)°C prior to use.

### (III) Staining Procedure for Manual Use Procedural Notes

The user should read these instructions carefully and become familiar with all components prior to use (see Precautions).

All reagents should be equilibrated to room temperature (20-25°C) prior to immunostaining. Likewise, all incubations should be performed at room temperature.

Do not allow tissue sections to dry during the staining procedure. Dried tissue sections may display increased nonspecific staining. To avoid drying place slides in a humid chamber.

### Deparaffinization and Rehydration

Prior to staining, tissue slides must be deparaffinized to remove embedding medium and rehydrated. Avoid incomplete removal of paraffin. Residual embedding medium will result in increased nonspecific staining.

STEP 1. Place slides in a xylene bath and incubate for 5 (±1) minutes. Change baths and repeat once.

STEP 2. Tap off excess liquid and place slides in absolute ethanol for 3 (±1) minutes. Change baths and repeat once.

STEP 3. Tap off excess liquid and place slides in 95% ethanol for 3 (±1) minutes. Change baths and repeat once.

STEP 4. Tap off excess liquid and place slides in reagent-quality water for 5 (±1) minutes.

STEP 5. Tap off excess liquid and place slides in Wash Buffer. Begin assay as outlined in Staining Procedure. Xylene and alcohol solutions should be changed after 40 slides. Toluene or xylene substitutes, such as Histoclear, may be used in place of xylene.

### Target Retrieval - Recommended Procedure: Water Bath

STEP 1. Fill staining jars, e.g., Coplin jars, with the diluted Target Retrieval Solution (see Reagent preparation). Place staining jars containing Target Retrieval Solution in water bath. Heat water bath and the Target Retrieval Solution to 95-99°C (do not boil). Cover jars with lids to stabilize the temperature and avoid evaporation.

STEP 2. Immerse room temperature deparaffinized sections in the preheated Target Retrieval Solution in the staining jars. Re-equilibrate temperature of the water bath and the Target Retrieval Solution back to 95-99°C. Incubate for 20 (±1) minutes at 95-99°C.

STEP 3. Remove the entire jar with slides from the water bath. Allow the slides to cool in the Target Retrieval Solution for 20 (±1) minutes at room temperature.

STEP 4. Decant Target Retrieval Solution and rinse sections in Wash Buffer (see Reagent Preparation).

STEP 5. For optimal performance, soak sections in Wash Buffer for 5 (±1) minutes after target retrieval and prior to staining.

### (IV) Manual Staining Protocol

STEP 1. Dual Endogenous Enzyme Block (code S2003). Tap off excess water. Using a lintless tissue, carefully wipe around the specimen to remove any remaining liquid and to keep reagent within the prescribed area. Apply enough Dual Endogenous Enzyme Block to cover specimen [minimum 3 drops (100µL)]. Incubate 5 (±1) minutes. Rinse gently with Wash Buffer from a wash bottle (do not focus stream directly on tissue or tissue may be washed off of slide). Place in a fresh Wash Buffer bath for 5 (±1) minutes.

STEP 2. Primary Antibody or Negative Control Reagent. Place slides in a humid chamber during the Primary Antibody/Negative Control Reagent and Labeled Polymer incubations to avoid drying of tissues. Tap off excess buffer and wipe slides as before. Apply enough Primary Antibody or Negative Control Reagent to cover specimen [minimum 3 drops (100 µL)]. Incubate 30 (±1) minutes in a humid chamber. Rinse slides as in Step 1.

STEP 3. EnVision + HRP, Anti-Rabbit (code K4002 or K4003). Tap off excess buffer and wipe slides as before. Apply enough Labelled Polymer to cover specimen [minimum 3 drops (100 µL)]. Incubate 30 (±1) minutes in a humid chamber. Rinse slides as in Step 1.

STEP 4. DAB+ Substrate-Chromogen Solution (code K3467 or K3468). Tap off excess buffer and wipe slides as before. Apply enough prepared DAB+ Substrate-Chromogen Solution to cover specimen [minimum 3 drops (100 µL)]. Incubate 10 (±1) minutes. Rinse gently with reagent-quality water from a wash bottle (do not focus flow directly on tissue or tissue may be washed off of slide). Collect DAB+ Substrate-Chromogen Solution waste in a hazardous materials container for proper disposal. Place in a reagent-quality water bath for 2-5 minutes. Proceed to **Counterstain and Mounting.**

### (V) Counterstain (Instructions for Hematoxylin)

The colored end-product of the staining reaction is alcohol and water insoluble. Hematoxylin, either alcohol or water-based such as DakoCytomation Hematoxylin (code S3301, automated; or S3302, manual), may be used. Do not use regressive counterstains.

STEP 1. Immerse slides in a bath of hematoxylin. Incubate for 2-5 minutes, depending on the strength of hematoxylin used.

STEP 2. Rinse gently in a reagent-quality water bath. Ensure that all residual hematoxylin has been cleared.

**Note:** The use of DakoCytomation's Hematoxylin (code S3302) is strongly recommended. Using a 3-minute incubation, this counterstain produces a mild purple/blue end product that does not obscure specific immunostaining. Strong counterstaining may mask weak c-kit/CD117 expression.

STEP 3. Rinse gently in a reagent-quality water bath for 2-5 minutes.

### (VI) Mounting

Non-aqueous, permanent mounting media such as DakoCytomation Ultramount (code S1964) is recommended. Aqueous mounting media such as DakoCytomation Faramount Mounting Medium, Ready-to-use (code S3025) or DakoCytomation GLYCERGEL® Mounting Medium (code C0563) is also suitable. Liquefy GLYCERGEL® by warming to approximately 40(±5)°C prior to use.

**Note:** It is recommended that slides are read within six weeks of staining. However, some fading may occur, depending on several factors including, but not limited to; counterstaining, mounting materials and methods and slide storage conditions. To minimize fading, store slides in the dark at room temperature (20-25°C).

Other methods of IHC are known in the art. For example, the following methods are incorporated herein in their entirety:
1. J.A. Ramos-Vara, "Technical Aspects of Immunohistochemistry", Vet Pathol., 42: 405-426 (2005).
2. J.T. Jørgensen et al., "Pharmacodiagnostics and targeted therapies - a rational approach for individualizing medical anticancer therapy in breast cancer", The Oncologist, 12(4):397-405 (April 2007). United States: AlphaMed Press. doi: 10.1634/theoncologist. 12-4-397. ISSN 1083-7159. PMID 17470682. Retrieved on 2008-03-14.
3. J.S. Gold et al., "Combined surgical and molecular therapy: the gastrointestinal stromal tumor model", Annals of Surgery, 244(2):176-184 (August 2006). United States: Lippincott Williams & Wilkins. doi:10.097/01.sla.0000218080.94145.cf..ISSN 0003-4932. PMID 16858179. Retrieved on 2008-03-14.
4. P.M. Harari, "Epidermal growth factor receptor inhibition strategies in oncology", Endocrine-Related Cancer, 11(4):689-708. (December 2004). England: Society for Endocrinology. doi:10.1677/erc.1.000600. ISSN 1351-0088. PMID 15613446. Retrieved on 2008-03-14.
5. M.F. Press et al., "Diagnostic evaluation of HER-2 as a molecular target: an assessment of accuracy and reproducibility of laboratory testing in large, prospective, randomized clinical trials", Clinical Cancer Research, 11(18):6598-6607 (September 15, 2005). United States: American Association for Cancer Research.. doi:10.1158/1078-0432.CCR-05-0636. ISSN 1078-0432. PMID 16166438. Retrieved on 2008-03-14.
6. F. Bibeau et al., "Assessment of epidermal growth factor receptor (EGFR) expression in primary colorectal carcinomas and their related metastases on tissue sections and tissue microarray", Virchows Archiv., 449(3):281-287 (July 25, 2006).

### EXAMPLE 9 - METHOD OF MEASURING THE LEVEL OF FGF2 EXPRESSION USING QUANTITATIVE RT-PCR ASSAYS

As outlined herein, FGF2(+) cells are sensitive to FGF2 inhibitors and dual FGF2/VEGFR2 inhibitors. Methods of ascertaining whether a cell is FGF2(+) are well known in the art. One method is to use RT-FCR to determine whether cells express FGF2. Briefly:

RNA quantification is performed using the TAQMAN® real-time-PCR fluorogenic assay. The TAQMAN® assay is one of the most precise methods for assaying the concentration of nucleic acid templates.

Total RNA from tissues is isolated using the TRIZOL® protocol (Invitrogen) and quantified by determining its absorbance at 260nM. An assessment of the 18s and 28s ribosomal RNA bands can be made by denaturing gel electrophoresis to determine RNA integrity.

RNA is prepared using standard methods, preferably, employing the RNEASY® Maxi Kit commercially available from Qiagen (Valencia, CA). A cDNA template for real-time PCR can be generated using the SUPERSCRIPT® First Strand Synthesis system for RT-PCR. Representative forward and reverse RT-PCT primers for FGF2 may be used. For example, FGF2 TAQMAN® primers from ABI may be used (Catalog No. Hs00266645_ml, or Catalog No. Hs00960934_ml; ABI).

SYBR® Green real-time PCR reactions are prepared as follows: The reaction mix contains 20 ng first strand cDNA; 50 nM Forward Primer; 50 nM Reverse Primer; 0.75X SYBR® Green I (Sigma); 1X SYBR® Green PCR Buffer (50 mM Tris-HCl pH 8.3, 75 mM KCl); 10% DMSO; 3 mM MgCl₂; 300 µM each dATP, dGTP, dTTP, dCTP; 1 U PLATINUM® Taq DNA Polymerase High Fidelity (Cat# 11304-029; Life Technologies; Rockville, MD). Real-time PCR is performed using an Applied Biosystems 5700 Sequence Detection System. Conditions are 95°C for 10 minutes (denaturation and activation of PLATINUM® Taq DNA Polymerase), 40 cycles of PCR (95°C for 15 seconds, 60°C for 1 minute). PCR products are analyzed for uniform melting using an analysis algorithm built into the 5700 Sequence Detection System.

cDNA quantification used in the normalization of template quantity is performed using TAQMAN® technology. TAQMAN® reactions are prepared as follows: The reaction mix comprises 20 ng first strand cDNA; 25 nM GAPDH-F3, Forward Primer; 250 nM GAPDH-R1 Reverse Primer; 200 nM GAPDH-PVIC TAQMAN® Probe (fluorescent dye labeled oligonucleotide primer); 1X Buffer A (Applied Biosystems); 5.5 mM MgCl₂; 300 µM dATP, dGTP, dTTP, dCTP; and 1 U AMPLITAQ GOLD® (Applied Biosystems). GAPDH (D-glyceraldehyde-3-phosphate dehydrogenase) is used as a control to normalize mRNA levels. Real-time TAQMAN® PCR is performed using an Applied Biosystems 7700 Sequence Detection System. Conditions are 95°C for 10 minutes (denaturation and activation of AMPLITAQ GOLD®), 40 cycles of PCR (95°C for 15 seconds, 60°C for 1 minute).

The sequences for the GAPDH oligonucleotides used in the TAQMAN® reactions are as follows:
GAPDH-F3: 5'-AGCCGAGCCACATCGCT-3' (SEQ ID NO:1);
GAPDH-R1: 5'-GTGACCAGGCGCCCAATAC-3' (SEQ ID NO:2); and
GAPDH-PVIC TAQMAN® Probe -VIC-5'-CAAATCCGTTGACTCCGACCTTCACCTT-3' TAMRA (SEQ ID NO:3).

The Sequence Detection System generates a Ct (threshold cycle) value that is used to calculate a concentration for each input cDNA template. cDNA levels for FGF2 are normalized to GAPDH cDNA levels to compensate for variations in total cDNA quantity in the input sample. This is done by generating GAPDH Ct values for each cell line. Ct values for the polynucleotide of interest and GAPDH are inserted into a modified version of the δδCt equation (Applied Biosystems PRISM® 7700 Sequence Detection System User Bulletin #2), which is used to calculate a GAPDH normalized relative cDNA level for each specific cDNA. The δδCt equation is as follows: relative quantity of nucleic acid template =2^{δδCt} = 2^{(δCta-δCtb)}, where δCta = Ct target - Ct GAPDH, and δCtb = Ct reference - Ct GAPDH. (No reference cell line is used for the calculation of relative quantity; 8Ctb is defined as 21).

It will be clear that the invention may be practiced otherwise than as particularly described in the foregoing description and examples. Numerous modifications and variations of the present invention are possible in light of the above teachings and, therefore, are within the scope of the appended claims.

The entire disclosure of each document cited (including patents, patent applications, journal articles, abstracts, laboratory manuals, books, GENBANK® Accession numbers, SWISS-PROT® Accession numbers, or other disclosures) in the Background of the Invention, Detailed Description, Brief Description of the Figures, and Examples is hereby incorporated herein by reference in their entirety. Further, the hard copy of the Sequence Listing submitted herewith, in addition to its corresponding Computer Readable Form, are incorporated herein by reference in their entireties.
The present invention further relates to the following embodiments:
1. A method for predicting the likelihood a patient will respond therapeutically to a cancer treatment comprising the administration of a VEGFR-2 modulator, wherein said prediction method comprises the steps of:
   (a) measuring the level of FGF2 in a sample from said patient; and
   (b) comparing the level of FGF2 in said sample relative to a standard to permit assignment of said sample to either being a member of an FGF2 positive class or an FGF2 negative class,
   wherein an FGF2 positive sample member indicates an increased likelihood the patient will respond therapeutically to said cancer treatment, whereas an FGF2 negative sample member indicates a decreased likelihood the patient will respond therapeutically to said cancer treatment.
2. The method according to embodiment 1, wherein said cancer treatment comprises a dual VEGFR-2 / FGFR-1 modulator.
3. The method according to embodiment 2, wherein said cancer treatment comprises the administration of [(1*R*),2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester, or a pharmaceutically acceptable salt, hydrate, or solvate thereof.
4. A method for treating a patient with cancer comprising the steps of:
   (a) measuring the level of FGF2 in a sample from said patient; and
   (b) comparing the level of FGF2 in said sample relative to a standard to permit assignment of said sample to either being an FGF2 positive or FGF2 negative sample,
   wherein an FGF2 positive sample indicates said patient should be administered a cancer treatment comprising a therapeutically acceptable amount of a VEGFR-2 modulator.
5. A kit for use in treating a patient with cancer, comprising:
   (a) a means for determining whether a sample from said patient is FGF2 positive;
   (b) a therapeutically effective amount of a treatment selected from the group consisting of: (i) a VEGFR-2 modulator; (ii) a dual VEGFR-2 / FGFR-1 modulator; and (iii) [(1*R*),2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester, or a pharmaceutically acceptable salt, hydrate, or solvate thereof, or a pharmaceutically acceptable salt or hydrate or solvate thereof; and
   (c) instructions for use of said kit.
6. A method for predicting the likelihood a patient will respond therapeutically to a cancer treatment comprising the administration of a VEGFR-2 modulator, wherein said method comprises the steps of:
   (a) measuring the level of FGF2 in a sample from said patient; and
   (b) comparing the level of FGF2 in said sample relative to a standard,
   wherein an elevated expression level of FGF2 in said sample relative to said standard indicates an increased likelihood that the patient will respond therapeutically to said cancer treatment, whereas a decreased or lower level of FGF2 in said sample relative to said standard indicates a decreased likelihood that the patient will respond therapeutically to said cancer treatment.
7. A method for treating a patient with cancer comprising the steps of:
   (a) measuring the level of FGF2 in a sample from said patient; and
   (b) comparing the level of FGF2 in said sample relative to a standard,
   wherein an elevated expression level of FGF2 in said sample relative to said standard indicates said patient should be administered a cancer treatment comprising a therapeutically acceptable amount of a VEGFR-2 modulator.
8. A kit for use in treating a patient with cancer, comprising:
   (a) a means for determining whether a sample from said patient has an elevated expression level of FGF2 in said sample relative to a standard;
   (b) a therapeutically effective amount of a treatment selected from the group consisting of: (i) a VEGFR-2 modulator; (ii) a dual VEGFR-2 / FGFR-1 modulator; and (iii) [(1*R*),2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester, or a pharmaceutically acceptable salt, hydrate, or solvate thereof, or a pharmaceutically acceptable salt or hydrate or solvate thereof; and
   (c) instructions for use of said kit.
9. A method of determining whether a patient suffering from cancer has received an efficacious dose of a cancer treatment comprising the administration of a VEGFR-2 modulator comprising the steps of:
   (a) measuring the level of Collagen IV in a sample from said patient; and
   (b) comparing the level of Collagen IV in said sample relative to a standard,
   wherein a decreased level of Collagen IV in said sample relative to said standard indicates an increased likelihood that the patient is receiving a therapeutically efficacious dose of said cancer treatment, whereas an increased or unchanged level of Collagen IV in said sample relative to said standard indicates a decreased likelihood that said patient has received a therapeutically efficacious dose of said cancer treatment.
10. The method according to embodiments 4, 6, 7 or 9 wherein said cancer treatment comprises a dual VEGFR-2 / FGFR-1 modulator.
11. The method according to embodiment 10, wherein said cancer treatment comprises the administration of [(1*R*),2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester, or a pharmaceutically acceptable salt, hydrate, or solvate thereof.
12. The method according to embodiments 1 , 4, 6, 7, or 9, wherein said measurement is performed using a method selected from the group consisting of: (a) PCR; (b) RT-PCR; (c) FISH; (d) IHC; (e) immuno-detection methods; (f) Western Blot; (g) ELISA; (h) radioimmuno assays; (i) immunoprecipitation ; (j) PET imaging; (k) HPLC; (1) surface plasmon resonance; (m) optical spectroscopy; and (i) mass spectrometry.

## Claims

1. A method for predicting the likelihood a patient will respond therapeutically to a cancer treatment comprising the administration of a VEGFR-2 modulator, wherein said prediction method comprises the steps of:
(a) measuring the level of FGFR1 protein or mRNA in a sample from said patient; and
(b) comparing the level of FGFR1 protein or mRNA in said sample relative to a standard to permit assignment of said sample to either being a member of an FGFR1 positive class or an FGFR1 negative class,
wherein an FGFR1 positive sample member indicates an increased likelihood the patient will respond therapeutically to said cancer treatment, whereas an FGFR1 negative sample member indicates a decreased likelihood the patient will respond therapeutically to said cancer treatment, and wherein said cancer treatment comprises the administration of [(1*R*),2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester, or a pharmaceutically acceptable salt, hydrate, or solvate thereof.

2. An *in vitro* diagnostic assay for use in designating a treatment regimen for a patient with cancer comprising the steps of:
(a) measuring the level of FGFR1 protein or mRNA in a sample from said patient; and
(b) comparing the level of FGFR1 protein or mRNA in said sample relative to a standard to permit assignment of said sample to either being an FGFR1 positive or FGFR1 negative sample,
wherein an FGFR1 positive sample indicates said patient should be administered a cancer treatment comprising a therapeutically acceptable amount of a VEGFR-2 modulator.

3. A kit for use in treating a patient with cancer, comprising:
(a) a means for determining whether a sample from said patient is FGFR1 positive;
(b) a therapeutically effective amount of a treatment selected from the group consisting of: (i) a VEGFR-2 modulator; (ii) a dual VEGFR-2 / FGFR-1 modulator; and (iii) [(1*R*),2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester, or a pharmaceutically acceptable salt, hydrate, or solvate thereof, or a pharmaceutically acceptable salt or hydrate or solvate thereof; and
(c) instructions for use of said kit.

4. A method for predicting the likelihood a patient will respond therapeutically to a cancer treatment comprising the administration of a VEGFR-2 modulator, wherein said method comprises the steps of:
(a) measuring the level of FGFR1 protein or mRNA in a sample from said patient; and
(b) comparing the level of FGFR1 protein or mRNA in said sample relative to a standard,
wherein an elevated expression level of FGFR1 in said sample relative to said standard indicates an increased likelihood that the patient will respond therapeutically to said cancer treatment, whereas a decreased or lower level of FGFR1 in said sample relative to said standard indicates a decreased likelihood that the patient will respond therapeutically to said cancer treatment.

5. A method for preceding the likelihood a patient will respond therapeutically to a cancer treatment comprising the administration of a VEGFR-2 modulator, wherein said prediction method comprises the steps of:
(a) measuring the level of VEGF protein or mRNA in a sample from said patient; and
(b) comparing the level of VEGF protein or mRNA in said sample relative to a standard to permit assignment of said sample to either being a member of an VEGFR2 positive class or an VEGFR2 negative class,
wherein an VEGF positive sample member indicates a decreased likelihood the patient will respond therapeutically to said cancer treatment, whereas a VEGF negative sample member indicates an increased likelihood the patient will respond therapeutically to said cancer treatment, and wherein said cancer treatment comprises the administration of [(1*R*),2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester, or a pharmaceutically acceptable salt, hydrate, or solvate thereof.

6. *An in vitro* diagnostic assay for use in designating a treatment regimen for a patient with cancer comprising the steps of:
(a) measuring the level of VEGF protein or mRNA in a sample from said patient; and
(b) comparing the level of VEGF protein or mRNA in said sample relative to a standard to permit assignment of said sample to either being an VEGF positive or VEGF negative sample,
wherein a VEGF negative sample indicates said patient should be administered a cancer treatment comprising a therapeutically acceptable amount of a VEGFR-2 modulator.

7. A kit for use in treating a patient with cancer, comprising:
(a) a means for determining whether a sample from said patient is VEGF positive;
(b) a therapeutically effective amount of a treatment selected from the group consisting of: (i) a VEGFR-2 modulator; (ii) a dual VEGFR-2 /FGFR-1 modulator; and (iii) [(1*R*),2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester, or a pharmaceutically acceptable salt, hydrate, or solvate thereof, or a pharmaceutically acceptable salt or hydrate or solvate thereof; and
(c) instructions for use of said kit.

8. A method for predicting the likelihood a patient will respond therapeutically to a cancer treatment comprising the administration of a VEGFR-2 modulator, wherein said method comprises the steps of:
(a) measuring the level of VEGF protein or mRNA in a sample from said patient; and
(b) comparing the level of VEGF protein or mRNA in said sample relative to a standard,
wherein an elevated expression level of VEGF in said sample relative to said standard indicates a decreased likelihood that the patient will respond therapeutically to said cancer treatment, whereas an increased or higher level of VEGF in said sample relative to said standard indicates a decreased likelihood that the patient will respond therapeutically to said cancer treatment.

9. A method for predicting the likelihood a patient will respond therapeutically to a cancer treatment comprising the administration of a VEGFR-2 modulator, wherein said prediction method comprises the steps of:
(a) administering a dual VEGFR-2 / FGFR-1 modulator;
(b) measuring the level of COL(IV) protein or mRNA in a sample from said patient; and
(c) comparing the level of COL(IV) protein or mRNA in said sample relative to a standard,
wherein an elevated expression level of COL(IV) in said sample relative to said standard indicates a decreased likelihood said patient will respond therapeutically to said cancer treatment, whereas a decreased or lower level of COL(IV) in said sample relative to said standard indicates an increased likelihood that the patient will respond therapeutically to said cancer treatment.

10. An *in vitro* diagnostic assay for use in designating a treatment regimen for a patient with cancer comprising the steps of:
(a) administering a dual VEGFR-2 / FGFR-1 modulator;
(b) measuring the level of COL(IV) protein or mRNA in a sample from said patient; and
(c) comparing the level of COL(IV) protein or mRNA in said sample relative to a standard,
wherein an elevated expression level of COL(IV) in said sample relative to said standard indicates a decreased likelihood said patient will respond therapeutically to said cancer treatment, whereas a decreased or lower level of COL(IV) in said sample relative to said standard indicates an increased likelihood that the patient will respond therapeutically to said cancer treatment.

11. A kit for use in treating a patient with cancer, comprising:
(a) a means for determining whether a sample from said patient is COL(IV) positive;
(b) a therapeutically effective amount of a treatment selected from the group consisting of: (i) a VEGFR-2 modulator; (ii) a dual VEGFR-2 /FGFR-2 modulator; and (iii) [(1*R*),2*S*]-2-Aminopropionic acid 2-[4-(4-fluoro-2-methyl-1H-indol-5-yloxy)-5-methylpyrrolo[2,1-f][1,2,4]triazin-6-yloxy]-1-methylethyl ester, or a pharmaceutically acceptable salt, hydrate or solvate thereof, or a pharmaceutically acceptable salt or hydrate or solvate thereof; and
(c) instructions for use of said kit.

12. A method for predicting the likelihood a patient will respond therapeutically to a cancer treatment comprising the administration of a VEGFR-2 modulator, wherein said prediction method comprises the steps of:
(a) administering a dual VEGFR-2 / FGFR-2 modulator;
(b) measuring the level of VEGFR2 protein or mRNA in a sample from said patient; and
(c) comparing the level of VEGFR2 protein or mRNA in said sample relative to a standard,
wherein an elevated expression level of VEGFR2 in said sample relative to said standard indicates an increased likelihood said patient will respond therapeutically to said cancer treatment, whereas a decreased or lower level of VEGFR2 in said sample relative to said standard indicates a decreased likelihood that the patient will respond therapeutically to said cancer treatment.

13. An *in vitro* diagnostic assay for use in designating a treatment regimen for a patient with cancer comprising the steps of:
(a) measuring the level of VEGFR2 protein or mRNA in a sample from said patient; and
(b) comparing the level of VEGFR2 protein or mRNA in said sample relative to a standard to permit assignment of said sample to either being a VEGFR2 positive or a VEGFR2 negative sample,
wherein a VEGFR2 positive sample indicates said patient should be administered a cancer treatment comprising a therapeutically acceptable amount of a VEGFR-2 modulator.

14. A kit for use in treating a patient with cancer, comprising:
(a) a means for determining whether a sample from said patient is VEGFR-2 positive;
(b) a therapeutically effective amount of a treatment selected from the group consisting of: (i) VEGFR-2 modulator; (ii) a dual VEGFR-2 / FGFR-1 modulator; and (iii) [(1*R*),2*S*]-2-Aminopropionic acid 2-[4-(4-fluoro-2-methyl-1 H-indol-5-yloxy)-5-methylpyrrolo[2,1-f][1,2,4]triazin-6-yloxy]-1-methylethyl ester, or a pharmaceutically acceptable salt, hydrate, or solvate thereof, or a pharmaceutically acceptable salt or hydrate or solvate thereof; and
(c) instructions for use of said kit.


SEQUENCE LISTING

<110>  Bristol-Myers Squibb Company

<120>  BIOMARKERS AND METHODS FOR DETERMINING SENSITIVITY TO VASCULAR
       ENDOTHELIAL GROWTH FACTOR RECEPTOR-2 MODULATORS

<130>  M/50218-EP-DIV

<150>  US 60/911,547
<151>  2007-04-13

<160>  3

<170>  PatentIn version 3.5

<210>  1
<211>  17
<212>  DNA
<213>  Homo sapiens

<400>  1
agccgagcca catcgct                                                    17


<210>  2
<211>  19
<212>  DNA
<213>  Homo sapiens

<400>  2
gtgaccaggc gcccaatac                                                  19


<210>  3
<211>  28
<212>  DNA
<213>  Homo sapiens

<400>  3
caaatccgtt gactccgacc ttcacctt                                        28